(19) Europäisches Patentamt
European Patent Office
Office européen des brevets

(11) EP 2 951 245 B1

(12) EUROPÄISCHE PATENTSCHRIFT

(45) Veröffentlichungstag und Bekanntmachung des Hinweises auf die Patenterteilung:
**02.08.2017 Patentblatt 2017/31**

(21) Anmeldenummer: **14704539.7**

(22) Anmeldetag: **31.01.2014**

(51) Int Cl.:
***C08F 299/02*** *(2006.01)* ***C09D 5/14*** *(2006.01)*
***C08L 67/06*** *(2006.01)*

(86) Internationale Anmeldenummer:
**PCT/EP2014/051880**

(87) Internationale Veröffentlichungsnummer:
**WO 2014/118314 (07.08.2014 Gazette 2014/32)**

# (54) VERNETZTER KUNSTSTOFFWERKSTOFF MIT INTRINSISCH ANTIMIKROBIELLER WIRKUNG AUF BASIS UNGESÄTTIGTER POLYESTER

CROSSLINKED PLASTIC MATERIAL WITH INTRINSIC ANTIMICROBIAL EFFECT ON THE BASIS OF UNSATURATED POLYESTER

MATIÈRE PREMIÈRE SYNTHÉTIQUE MISE EN RÉSEAU AVEC ACTION ANTIMICROBIENNE INTRINSÈQUE À BASE DE POLYESTER INSATURÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.01.2013 EP 13000472**

(43) Veröffentlichungstag der Anmeldung:
**09.12.2015 Patentblatt 2015/50**

(73) Patentinhaber: **Fachhochschule Münster**
**48565 Steinfurt (DE)**

(72) Erfinder:
- **LORENZ, Reinhard**
**48565 Steinfurt (DE)**
- **FISCHER, Björn**
**48369 Saerbeck (DE)**
- **KREYENSCHMIDT, Martin**
**49393 Lohne (DE)**
- **KREYENSCHMIDT, Judith**
**53359 Rheinbach (DE)**

(74) Vertreter: **Ter Meer Steinmeister & Partner**
**Patentanwälte mbB**
**Nymphenburger Straße 4**
**80335 München (DE)**

(56) Entgegenhaltungen:
**DE-A1-102007 054 133**

EP 2 951 245 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft eine radikalisch härtbare chemische Zusammensetzung in Form eines Harzes zur Herstellung von Werkstoffen mit intrinsisch antimikrobieller Wirkung sowie die Anwendungen dieser Werkstoffe sowie ein Verfahren zur Herstellung dieser Harze und Werkstoffe sowie die Verwendung aminofunktionalisierter Styrolderivate als Reaktivverdünner.

Bei zahlreichen Anwendungen von Kunststoffen und Werkstoffen ist der Aufwuchs von Algen, Pilzen oder Muscheln eine unerfreuliche Begleiterscheinung. Deutlich ernstere Probleme können allerdings durch die Bildung von bakteriellen Biofilmen und die Übertragung pathogener Keime entstehen. Der Bekämpfung und Vermeidung dieser unerwünschten Effekte kommt somit große medizinische, hygienische, lebensmitteltechnologische und praktische Bedeutung zu: Man behilft sich im Allgemeinen mit mechanisch-chemischer Reinigung, mit Additivierung des Kunststoffs oder Lackes durch Zugabe klassischer Biozide (z.B. Silber-, Kupfer- oder Zinkverbindungen oder organische Verbindungen wie Triclosan, 10,10'-Oxy-bis-phenoxyarsin, N-(Trifluormethylthio)phthalimid, N-(Trichlormethylthio)-phthalimid, verschiedene Isothiazolione) sowie mit fortgesetzter Verwendung verschiedener äußerer Desinfektionsmittel - wie es beispielsweise in Krankenhäusern oder auch in der Lebensmittel- und Fleischverarbeitung gängige Praxis ist.

Es war bislang schwierig, Oberflächen antimikrobiell auszustatten, ohne dass der als Additiv zugegebene antimikrobielle Wirkstoff aus der Oberfläche herausdiffundiert. Es gab einige Versuche, antimikrobiell funktionalisierte Monomere zum Aufbau von antimikrobiellen Polymeren zu nutzen. Eines dieser Monomere war beispielsweise tert.-Butylaminoethylmethacrylat (TBAEMA). Der Nachteil dieser bislang verfügbaren Polymere, z.B. Poly(TBAEMA), lag darin, dass die Glasübergangstemperatur sehr niedrig lag, wie beispielsweise unterhalb von 50 °C bei Poly(TBAEMA).

US 6,242,526 beschreibt antimikrobielle Polymerlatices, die sich aus einer ethylenisch ungesättigten Säure als Anion und einer quaternären Ammoniumverbindung als Kation zusammensetzen, wobei die antimikrobielle Wirkung der quaternären Ammoniumverbindungen zuzuordnen ist.

US 4,810,567 beschreibt antimikrobielle Textilien, wobei verschiedene Basistextilien durch eine graft-Copolymerisation mit Säuregruppen enthaltenden Monomeren funktionalisiert werden, an die durch Amidierung antimikrobielle Proteine und Antibiotika gebunden werden.

US 5,614,568 beschreibt einen antibakteriellen, thermoplastischen Compound, der unter anderem Silber-, Kupfer- oder Zinkzeolithe enthält. Zudem werden polymere und niedermolekulare Additive mit speziellen funktionellen Gruppen verwendet, die die antimikrobielle Wirkung steigern.

US 4,447,580 beschreibt ein Copolymer auf Acrylat-Basis enthaltend aminofunktionalisierte Styrol-Monomereinheiten zur Herstellung von Tauchlacken für die Kataphorese. Diese Copolymere werden über eine thermisch aktivierte Michael-Addition mit oligomeren und polymeren Verbindungen umgesetzt, die aktivierte Doppelbindungen enthalten. Die aminofunktionalisierten Styrolderivate werden nicht für eine radikalische Vernetzung genutzt und auch nicht für eine antimikrobielle Wirkung.

JP 2000 239 281 A beschreibt die Synthese von aminofunktionalisierten Styrolderivaten, aus denen vernetzte Polymere hergestellt werden, die in immobilisierte polymere Lithiumamide überführt werden. Diese dienen der organischen Synthese.

US 6,200,680 beschreibt die Herstellung von Zinkoxid-Partikeln unter Verwendung polymerer Hilfsstoffe, darunter aminofunktionalisierte Polymere auf der Basis von aminofunktionalisierten Styrolderivaten.

US 4,021,416 beschreibt aminoethanthiol-funktionalisierte Styrolderivate, die als Komplexbildner für Silberionen und/oder lösliche Silberkomplexe in der Fotografie genutzt werden können.

Kuno et al. (Reactive & Functional Polymers 43 (2000) 43 - 51) beschreiben Poly[N-(p-vinylbenzyliden)-tert.-butylaminoxid] als neuen Radikalfänger für Anwendungen in der Umwelttechnologie.

DE 102 42 561 A1 offenbart eine antimikrobielle Beschichtung, wobei die antimikrobielle Beschichtung Polymere von speziellen cyclischen Aminen mit zumindest einer polymerisierbaren ungesättigten Gruppe aufweist. Weiterhin wird ein Verfahren zur Herstellung dieser antimikrobiellen Beschichtungen und deren Verwendung zur Herstellung von Erzeugnissen mit antimikrobiellen Eigenschaften beschrieben.

DE 44 32 985 A1 offenbart Bindemittel und deren Herstellung sowie deren Verwendung in Überzugsmitteln für kratz- und säurefeste Überzüge. Die Bindemittel werden durch eine radikalische Polymerisation von a) (Meth)acrylmonomer und gegebenenfalls weiterem radikalisch polymerisierbaren Monomer(en) in Gegenwart von b) Cycloolefinhomopolymer und/oder Cycloolefincopolymer, die frei von olefinischen Doppelbindungen sind, gewonnen.

DE 197 23 504 C1 offenbart Beschichtungsmittel insbesondere zur Beschichtung von Kunststoffen, Verfahren zu seiner Herstellung und die Verwendung als Deck- oder Klarlack. Die Beschichtungsmittel enthalten

a) ein oder mehrere spezielle Polyersterharze,

b) ein oder mehrere spezielle Polyacrylatharze,

c) ein oder mehrere Di- und/oder Polyisocyante

d) ein oder mehrere Lichtschutzmittel auf Basis eines UV-Absorbers

e) ein oder mehrere spezielle Lichtschutzmittel auf Basis sterisch gehinderter Amine, und

f) ein oder mehrere organische Lösungsmittel.

H. Menzel gibt in "Schutzschicht gegen Bakterien" (Nachrichten aus der Chemie, 59, November 2011, S. 1039-43) einen Überblick über medizinisch geeignete antimikrobielle Beschichtungen auf der Basis von Polymeren mit hydrophoben Strukturelementen in Kombination mit einer hohen positiven Ladungsdichte. Die verschiedenen Wirkmechanismen gegenüber gramm-positiven und gramm-negativen Bakterien werden kurz erörtert. Die meisten Strukturen beruhen auf quaternären Ammonium- und Phosphoniumionen. Antimikrobielle Polymere für kompakte werkstoffliche Anwendungen werden nicht behandelt.

Die Aufgabe der vorliegenden Erfindung besteht darin, eine in der Praxis leicht einsetzbare Technologie bereitzustellen, mit der antimikrobiell ausgerüstete Oberflächen und Erzeugnisse hergestellt werden können, bei denen kein antimikrobielles Additiv aus der Oberfläche oder den Erzeugnissen herausdiffundieren kann. Eine weitere Aufgabe besteht darin einen antimikrobiellen Werkstoff bereitzustellen, der "in the bulk", d.h. im Gesamtvolumen, antimikrobiell funktionalisiert ist und der daher nach Verletzung oder Veränderung seiner Oberfläche, z.B. durch Schlag, Abrasion oder Schnitt, eine neue Oberfläche bildet, die ebenfalls antimikrobiell ist.

Die der Erfindung zu Grunde liegende Aufgabe wird in einer ersten Ausführungsform gelöst durch eine UP-Harz-Zusammensetzung zur Herstellung von Erzeugnissen mit antimikrobieller Wirkung enthaltend

a) einen ungesättigten Polyester aus einerseits Dicarbonsäure und/oder Anhydrid und andererseits Diol im molaren Verhältnis 1,25:1 bis 0,75:1, wobei die Dicarbonsäure und/oder das Anhydrid wenigstens teilweise mit einer radikalisch reaktiven Doppelbindung funktionalisiert sind, und

b) Styrolderivat als Reaktivverdünner, wobei bevorzugt je Doppelbindung in Komponente a) 0,5 bis 8 Styrolderivatmoleküle in der UP-Harz-Zusammensetzung vorhanden sind, und

wobei wenigstens eine Entität ausgewählt aus Styrolderivat, Dicarbonsäure und/oder Diol aminofunktionalisiert ist, wobei die Aminofunktionaliät unter die Formel

$$-(CH_2)_q-NH_pR^1R^2A_p$$

fällt, wobei

q entweder 0, 1 oder 2 ist, wobei q ≠ 0 ist, sofern die Funktionalität an einen Aromaten gebunden ist,

p 0 oder 1 ist,

$R^1$ ausgewählt ist aus H, linearen oder verzweigten oder zyklischen Alkylresten aufweisend 1 bis 10 Kohlenstoffatome,

$R^2$ ein linearer oder verzweigter oder zyklischer Alkylrest aufweisend 1 bis 10 Kohlenstoffatome ist,

A das Anion einer Säure ist und,

der Amin-Stickstoff N der obenstehenden Formel neutral (p = 0) oder positiv (p = 1) geladen ist.

Der nach der Härtung dieser erfindungsgemäßen UP-Harz-Zusammensetzung gebildete vernetzte Kunststoffwerkstoff wirkt ohne Verwendung zusätzlicher Biozide intrinsisch antimikrobiell. Die neuen Werkstoffe werden daher als intrinsisch antimikrobiell bezeichnet. Der Vorteil der erfindungsgemäßen UP-Harz-Zusammensetzung ist, dass hiermit Erzeugnisse hergestellt werden können, aus denen antimikrobielle Wirkstoffe nicht austreten können und dass diese Erzeugnisse auch bei mechanischer Veränderung oder Beschädigung ihrer Oberfläche antimikrobiell bleiben.

Die UP-Harz-Zusammensetzung im Sinne der Erfindung enthält einen ungesättigten Polyester und wenigstens einen Reaktivverdünner.

Es zeigte sich, dass die der Erfindung zugrunde liegende Aufgabe mit radikalisch vernetzenden Harzen vom

Typ der ungesättigten Polyesterharze (UP-Harze) gelöst werden kann. Dazu wird der üblicherweise eingesetzte Reaktivverdünner Styrol durch ein aminofunktionalisiertes Styrolderivat, eine Mischung verschiedener aminofunktionalisierter Styrolderivate, eine Mischung eines oder mehrerer aminofunktionalisierter Styrolderivate mit einem oder mehreren aminofunktionalisierten Methacrylat(en) oder eine dieser Mischungen mit weiteren Reaktivverdünnern ersetzt. Zu den weiteren Reaktivverdünnern zählen beispielweise Styrol, Methylstyrol, Vinyltoluol, tert.-Butylstyrol, 4-Vinylpyridin, 3-Vinylpyridin, 2-Vinylpyridin, Methylmethacrylat, Divinylbenzol, 1,2,4-Trivinylcyclohexan, Diallylphthalat, Diallylisophthalat, Trisallylisocyanurat.

Zusätzlich oder alternativ kann der ungesättigte Polyester im UP-Harz mit Alkylamino- oder Dialkylaminogruppen antimikrobiell funktionalisiert werden. Weiterhin sind Mischungen dieser antimikrobiell ausgerüsteten UP-Harze mit intrinsisch antimikrobiellen VE (Vinylester)- und/oder VEU (Vinylesterurethan)-Harzen erfindungsgemäß möglich.

Darüber hinaus sind Mischungen mit thermoplastischen Polymeren und intrinsisch antimikrobiellen thermoplastischen Polymeren erfindungsgemäß, beispielsweise zur Zähmodifizierung oder zur Schwundkompensation der gebildeten Duromere. Auch sind Mischungen dieser antimikrobiell ausgerüsteten UP-Harze mit konventionellen, nicht antimikrobiell ausgerüsteten Harzen vom Typ der UP-Harze, VE-Harze, VEU-Harze und Methacrylatharze erfindungsgemäß möglich, soweit der antimikrobielle Effekt erhalten bleibt.

**Ungesättigter Polyester**

Der ungesättigte Polyester, der bei Raumtemperatur oft fest oder halbfest ist, wird beispielsweise durch Polykondensation in der Schmelze aus Dicarbonsäuren und Anhydriden, welche wenigstens teilweise mit einer radikalisch reaktiven Doppelbindung funktionalisiert sind, sowie Diolen hergestellt. Nach Abmischen des ungesättigten Polyesters mit dem Reaktivverdünner erhält man ein bei Raumtemperatur meist flüssiges Harz, das als ungesättigtes Polyesterharz (UP-Harz) bezeichnet wird. Nach der radikalischen Härtung (chemische Vernetzung) des UP-Harzes spricht man vom UP-Duromer oder UP-Netzwerk.

Nach der Polykondensation von einerseits Dicarbonsäure und/oder Anhydrid und andererseits Diol liegt der ungesättigte Polyester als eine Mischung von Polymer, Oligomer und restlichem Monomer entsprechend der jeweiligen Molmassenverteilung vor.

Dicarbonsäure, Anhydrid und Diol im Sinne der Erfindung können auch jeweils Mischungen verschiedener Dicarbonsäuren, Anhydride und Diole sein.

Das molare Verhältnis zwischen einerseits Dicarbonsäure und/oder Anhydrid und andererseits Diol liegt vorzugsweise in einem Bereich von 0,9:1 bis 1:0,9, besonders bevorzugt von 1,03:1 bis 0,97:1, um ungesättigte Polyester mit hinreichender Molmasse herzustellen. Auftretende Nebenreaktionen, die zur Bildung flüchtiger Nebenprodukte führen, können darüber hinaus die erforderliche Stöchiometrie stark beeinflussen. Zum Beispiel bei Verwendung von Butandiol-1,4 ist dies die Bildung von Tetrahydrofuran; bei Verwendung von 1,2-Propylenglykol ist es die Bildung von 2-Ethyl-4-methyl-1,3-dioxolan. Beide Nebenreaktionen können bewirken, dass ein deutlicher Überschuss bei der Kondensation erforderlich wird. Im Fall von 1,2-Propylenglykol sind dies vorzugsweise 7-15 mol-%, im Fall von 1,4-Butandiol vorzugsweise etwa 30 mol-%.

Bei den Dicarbonsäuren sind beispielsweise zwei Typen zu unterscheiden:

- Die radikalisch reaktiven Carbonsäuren, insbesondere Citracon-, Fumar-, Itacon-, Malein- und/oder Mesaconsäure, die nach der Polykondensation eine radikalische Vernetzung des ungesättigten Polyesters mit dem Reaktivverdünner möglich machen.

- Die nichtreaktiven Dicarbonsäuren nehmen lediglich an der Polykondensationsreaktion teil, nicht aber an der Vernetzungsreaktion. Hierzu zählen aromatische Dicarbonsäuren (Phthalsäure, Isophthalsäure, Terephthalsäure usw.), aliphatische Dicarbonsäuren (Bernsteinsäure, Adipinsäure, Sebacinsäure usw.) sowie cycloaliphatische Dicarbonsäuren (1,2-Cyclohexandicarbonsäure, 1,4-Cyclohexandicarbonsäure, 1,2,5,6-Tetrahydrophthalsäure usw.).

Das Anhydrid kann ein Anhydrid der vorgenannten Dicarbonsäuren sein. Die Mischung der Dicarbonsäuren kann bis zu 10% einer Tricarbonsäure oder einer Tetracarbonsäure enthalten, z.B. Trimellithsäure bzw. Trimellithsäureanhydrid oder Pyromellithsäure bzw. Pyromellithsäuredianhydrid.

Das Diol ist vorzugsweise ausgewählt aus der Gruppe 1,2- Ethylenglykol, 1,2-Propandiol, 1,3-Propandiol, Diethylenglykol, Dipropylenglykol, Triethylenglykol, Tripropylenglykol, Neopentylglykol, Butan-1,3-diol, Butan-1,2-diol, Butan-2,3-diol, Butan-1,4-diol, 2,2-Butylethylpropandiol-1,3, 2-Metylpropandiol-1,3, Pentan-1,5-diol, Hexan-1,6-diol, 2,4-Dimethyl-2-ethylhexan-1,3-diol, Hydroxypivalinsäureneopentylglykolester, Isosorbid, cycloaliphatische Diole (Tricyclodecandimethanol, perhydriertes Bisphenol A, 1,4-Cyclohexandimethanol, Norbornylenglykol, usw.), ethoxyliertes und propoxyliertes Bisphenol A oder Mischungen derselben.

Diol im Sinne der Erfindung kann auch eine Mischung verschiedener Diole sein, die bis höchstens 10 mol%

ein Triol enthalten kann. Das Triol ist vorzugsweise ausgewählt aus Glycerin, Trimethylolpropan, Triethanolamin, Triisopropanolamin oder Mischungen derselben. Anstelle des Triols kann auch eine andere trifunktionelle Verbindung eingesetzt werden, z.B. Dimethylolbuttersäure oder Dimethylolpropionsäure.

Dicyclopentadien kann mit Maleinsäure monofunktionell umgesetzt werden zum Dicyclopentadienyl-Maleinsäurehalbester, der sich in begrenzter Menge in ungesättigte Polyester einbauen lässt. Diese (sogenannte DCPD-Harze) können erfindungsgemäß ebenfalls antimikrobiell funktionalisiert werden.

**Reaktivverdünner**

Als Reaktivverdünner wird im Sinne der Erfindung in diesem Zusammenhang sowohl ein einzelnes geeignetes Monomer als auch eine geeignete Monomermischung bezeichnet. Entscheidend ist, dass das Monomer oder die Monomermischung eine radikalische Vernetzung erlauben und vollständig oder weitgehend vollständig ins Netzwerk eingebaut werden.

Die Zusammensetzung kann neben dem Styrolderivat b) einen weiteren oder weitere Reaktivverdünner aus der Gruppe der Styrolderivate und/oder Methacrylate und/oder höherfunktionellen Monomere beinhalten.

Das Verhältnis von Doppelbindung in Komponente a) zu Styrolderivatmolekül ist grundsätzlich zunächst nicht beschränkt. In einer bevorzugten Ausführungsform sind je Doppelbindung in Komponente a) 0,5 bis 8, bevorzugt 0,7 bis 7, mehr bevorzugt 1 bis 6, noch mehr bevorzugt 1,5 bis 4, insbesondere 2,0 bis 3,5 Styrolderivatmoleküle in der Zusammensetzung vorhanden. Liegt die Zahl darunter, so kann gegebenenfalls die Zusammensetzung zu hochviskos werden oder es kann zu ungenügender Vernetzung kommen. Liegt die Zahl darüber, so kann es sein, dass die Zusammensetzung gegebenenfalls zu niederviskos wird oder dass Styrolderivatmoleküle nicht vollständig bei der Vernetzungsreaktion reagieren und freies Restmonomer aus dem Erzeugnis oder der Oberfläche herausdiffundieren kann. Um dies zu verhindern, sollte der Restmonomergehalt vorzugsweise durch eine längere Nachhärtung bei erhöhter Temperatur abgesenkt werden.

**Aminofunktionalität**

Wenigstens eine Entität ausgewählt aus Styrolderivat, Dicarbonsäure und/oder Diol ist erfindungsgemäß aminofunktionalisiert,
wobei die Aminofunktionaliät unabhängig von der Entität unter die Formel

$$-(CH_2)_q-NH_pR^1R^2A_p$$

fällt, wobei

q entweder 0, 1 oder 2 ist, wobei $q \neq 0$ ist, sofern die Funktionalität an einen Aromaten gebunden ist,

p 0 oder 1 ist,

$R^1$ ausgewählt ist aus H, linearen oder verzweigten oder zyklischen Alkylresten aufweisend 1 bis 10 Kohlenstoffatome,

$R^2$ ein linearer oder verzweigter oder zyklischer Alkylrest aufweisend 1 bis 10 Kohlenstoffatome ist,

A das Anion einer Säure ist und,

der Amin-Stickstoff N der obenstehenden Formel neutral (p = 0) oder positiv (p = 1) geladen ist.

p kann bevorzugt nach der Neutralisation mit einer Säure HA gleich 1 sein.

$R^2$ weist vorzugsweise 1 bis 10 Kohlenstoffatome, bevorzugt 3 bis 5 Kohlenstoffatome auf.

$R^2$ ist bevorzugt verzweigt, insbesondere ausgewählt aus iso-Propyl, tert.-Butyl oder tert.-Pentyl.

$R^1$ ist vorzugsweise ausgewählt aus H und $R^2$.

Sofern $R^2$ mehr als drei Kohlenstoffatome aufweist, ist $R^1$ vorzugsweise ausgewählt aus H oder einem Alkylrest mit 1 bis 3 Kohlenstoffatomen.

Bei der Säure kann es sich um Kohlensäure handeln, die in Gegenwart von Umgebungsluft oder Wasser durch enthaltendes $CO_2$ in situ gebildet wird. In diesem Fall liegt eine Mischung aus neutralisiertem und nicht neutralisiertem Amin vor. Bei dem an der Umgebungsluft oder in Gegenwart von Wasser neutralisierten Amin kann das Gegenion A vor allem $HCO_3^-$ sein. Eine weitere Neutralisation kann sich durch die Reaktion mit COOH-Gruppen, die am Polymer oder Oligomer oder Restmonomer fixiert sind, ergeben. Die Gegenionen können in diesem Falle Polymer-$COO^-$, Oligomer-$COO^-$ und Restmonomer-COO sein. Auch die Zugabe und Anwendung anderer mono- oder höherfunktioneller Säuren ist möglich.

In einer bevorzugten Ausführungsform ist das Styrolderivat aminofunktionalisiert. Da die Styrolmonomere den ungesättigten Polyester in der Regel in einem breiten Zusammensetzungsbereich problemlos quer vernetzten, kann man über den Gehalt an aminofunktionalisiertem Reaktivverdünner besonders gut den Gehalt an Aminofunktionen im entstehenden vernetzten Kunststoff steuern.

In einer alternativen Ausführungsform sind der ungesättigte Polyester (bzw. das Diol und/oder die Dicarbonsäure) aminofunktionalisiert und/oder das Styrolderivat aminofunktionalisiert. Hierbei fällt die Aminofunktionalität unabhängig von der Entität unter die obengenannte Formel $-(CH_2)_qNH_pR^1R^2A_p$, d.h. dass beispielsweise der Polyester (bzw. Diol und/oder die Dicarbonsäure) eine andere Aminofunktionalität aufweisen kann als das Styrolderivat.

Ganz besonders bevorzugt ist nur das Styrolderivat aminofunktionalisiert.

**Aminofunktionalisierung des ungesättigten Polyesters**

Für die Funktionalisierung des ungesättigten Polyesters bieten sich vorzugsweise verschiedene Stufen im Laufe der Rohstoff- bzw. Harzherstellung an:

- Antimikrobielle Funktionalisierung der Rohstoffe und nachfolgende Kondensation des ungesättigten Polyesters. So können Diol und/oder Dicarbonsäuren des ungesättigten Polyesters funktionalisiert werden.
- Es kann eine antimikrobielle Funktionalisierung der ungesättigten Polyester vor der Abmischung mit dem Reaktivverdünner erfolgen.
- Es kann eine antimikrobielle Funktionalisierung von ungesättigten Polyestern nach dem vollständigen oder partiellen Abmischen mit dem Reaktivverdünner erfolgen.

Ziel der antimikrobiellen Funktionalisierung ist es, die Konzentration antimikrobieller Funktionen im UP-Duromer weiter zu erhöhen.

Die antimikrobiell wirksamen Aminogruppen können vorzugsweise vor der Herstellung des ungesättigten Polyesters in bestimmte halogenierte oder anderweitig vorfunktionalisierte Diole und/oder Dicarbonsäuren eingeführt werden, die dann zur Herstellung des ungesättigten Polyesters dienen. Als Beispiel sei hier die Synthese einer aminofunktionalisierten Säurekomponente, der 2,3-Bis(tert.-butylamino)butandisäure aus 2,3-Dibrombutandisäure und tert.-Butylamin durch Substitution genannt.

Eine Alternative zur Modifikation der Rohstoffe stellt die polymeranaloge Funktionalisierung dar. Die nachträgliche Funktionalisierung ist möglich, da bei der Herstellung des Polyesters neben Standardkomponenten auch halogenierte Diole und Dicarbonsäuren problemlos eingesetzt werden können, deren Verwendung Stand der Technik bei flammgeschützten Harzen ist. Als halogenierte Rohstoffe werden vorzugsweise kommerziell verfügbare Vertreter sowie durch Bromierung hergestellte Zwischenstufen verwendet. Nach erfolgter Herstellung des Polyesters wird dieser vorzugsweise in einem geeigneten Lösungsmittel gelöst und eine polymeranaloge Substitution mit Mono- oder Dialkylaminen durchgeführt. Als Beispiel sei hier die Kondensation eines ungesättigten Polyesters genannt, dessen Diolzusammensetzung zu Teilen Dibromneopentylglykol enthält. Die so eingeführten Bromsubstituenten können nach Aufnahme des fertig kondensierten Polyesters in einem geeigneten Lösungsmittel durch Substitution mit tert.-Butylamin polymeranalog umgesetzt werden. Beim Lösungsmittel handelt es sich vorzugsweise um den Reaktivverdünner.

Bei den ungesättigten Polyestern bietet sich zur polymeranalogen Reaktion außerdem die Michael-Addition von Mono- oder Dialkylaminen an Teile der Fumar- und Maleinester-Doppelbindungen zur antimikrobiellen Funktionalisierung an. Für diese Funktionalisierung werden vorzugsweise ungesättigte Polyester mit hohem Gehalt an Fumar- bzw. Maleinesterstrukturen genutzt und beispielsweise zwischen 1 und 80 mol% der reaktiven Doppelbindungen bei relativ niedriger Temperatur (RT bis 120 °C) mit entsprechenden Aminen umgesetzt. Die verbleibenden reaktiven Doppelbindungen können der radikalischen Vernetzung dienen.

Im Falle der ungesättigten Polyester besteht des Weiteren die Möglichkeit, die antimikrobiell wirksamen Aminogruppen in Form von diethoxylierten oder dipropoxylierten Monoalkylaminen einzubringen, die im ungesättigten Polyester als Diolkomponente eingesetzt werden können. Beispiele sind die Diole Methyldiethanolamin, tert.-Butyldiethanolamin, Methyldüsopropanolamin, tert.-Butyldüsopropanolamin oder N-Hydroxyethylpiperazin, Bishydroxyethylpiperazin, N-Hydroxypropylpiperazin, Bishydroxypropylpiperazin oder 3-(Diethylamino)-1,2-propandiol.

**Aminofunktionalisiertes Styrolderivat**

In der erfindungsgemäßen Zusammensetzung ist vorzugsweise das Styrolderivat aminofunktionalisiert. Das Molekulargewicht des aminofunktionalisierten Styrolderivats liegt vorzugsweise in einem Bereich von 100 bis 300 g/mol, insbesondere in einem Bereich von 170 bis 250 g/mol. Es hat sich herausgestellt, dass die Vernetzungsreaktion bei einem zu hohen Molekulargewicht des Monomers unvollständig ablaufen kann.

Das aminofunktionalisierte Styrolderivat weist vorzugsweise 10 bis 20 Kohlenstoffatome, insbesondere 12 bis 18 Kohlenstoffatome auf.

Das erfindungsgemäße aminofunktionalisierte Styrolderivat ist vorzugsweise ausgewählt aus der Gruppe N-(4-ethenylbenzyl)-2-methylpropan-2-amin (auch tert.-Butyl-Aminomethyl-Styrol oder TBAMS), N-(4-ethenylbenzyl)ethanamin (auch Ethyl-Aminomethyl-Styrol oder EAMS),N-(4-ethenylbenzyl)propan-1-amin (auch n-Propyl-Aminomethyl-Styrol oder PAMS), N-(4-ethenylbenzyl)propan-2-amin (auch iso-Propyl-Aminomethyl-Styrol oder IPAMS), N-(4-ethenylbenzyl)butan-1-amin (auch n-Butyl-Aminomethyl-Styrol oder BAMS), N-(4-ethenylbenzyl)butan-2-amin (auch sec-Butyl-Aminomethyl-Styrol oder SBAMS), N-(4-ethenylbenzyl)-2-methylpropan-1-amin (auch iso-Butyl-Aminomethyl-Styrol oder IBAMS), N-(4-ethenylbenzyl)pentan-1-amin (auch n-Pentyl-Aminomethyl-Styrol oder PENAMS), N-(4-ethenylbenzyl)-3-methylbutan-1-amin (auch iso-Pentyl-Aminomethyl-Styrol oder IPENAMS), N-(4-ethenylbenzyl)pentan-3-amin (auch 3-Pentyl-Aminomethyl-Styrol oder 3-PENAMS), N-(4-ethenylbenzyl)-2-methylbutan-2-amin (auch tert.-Pentyl-Aminomethyl-Styrol oder TPAMS), N-(4-ethenylbenzyl)cyclopentanamin (auch Cyclopentyl-Aminomethyl-Styrol oder CPENAMS), N-(4-ethenylbenzyl)cyclohexanamin (auch Cyclohexyl-Aminomethyl-Styrol oder CHAMS), N-(4-ethenylbenzyl)-N,N-dimethylamin (auch Dimethyl-Aminomethyl-Styrol oder DMAMS, N-(4-ethenylbenzyl)-N,N-diethylamin (auch Diethyl-Aminomethyl-Styrol oder DEAMS), N-(4-ethenylbenzyl)-N-(propan-2-yl)propan-2-amin (auch Diisopropyl-Aminomethyl-Styrol oder DIPAMS) und Mischungen daraus, wobei diese Verbindungen wie folgt abgebildet sind:

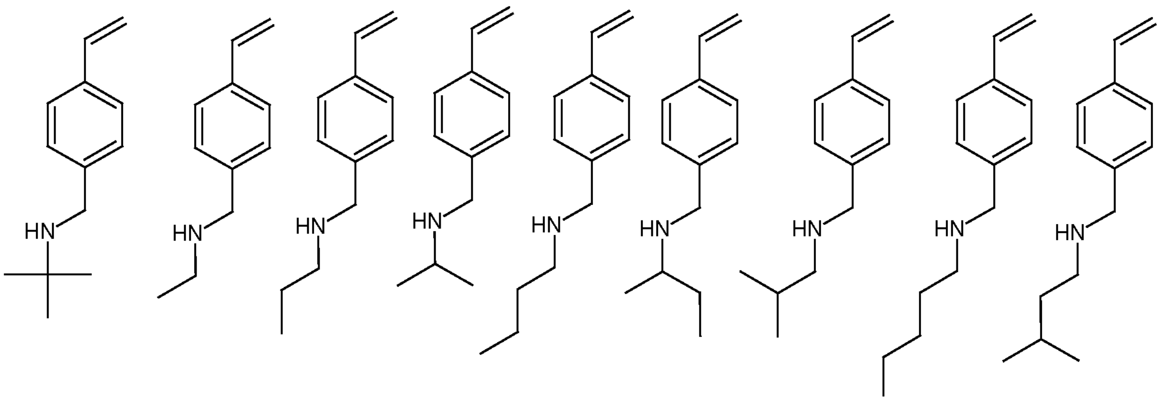

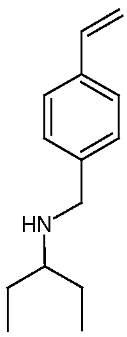

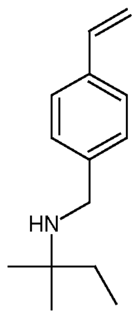

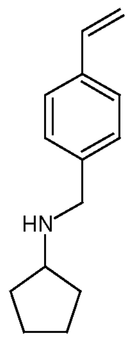

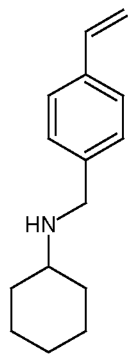

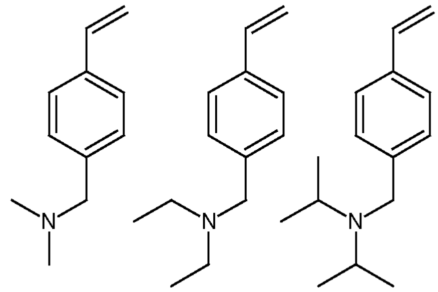

Neben den bereits genannten para-Isomeren können erfindungsgemäß auch alle meta-Isomere sowie alle ortho-Isomere der bereits genannten Derivate sowie beliebige Mischungen aller ortho-, meta- und para-Isomere verwendet werden.

In einer Ausführungsform wird die der Erfindung zu Grunde liegende Aufgabe auch gelöst durch Verwendung eines oder einer Mischung dieser drei Stoffe

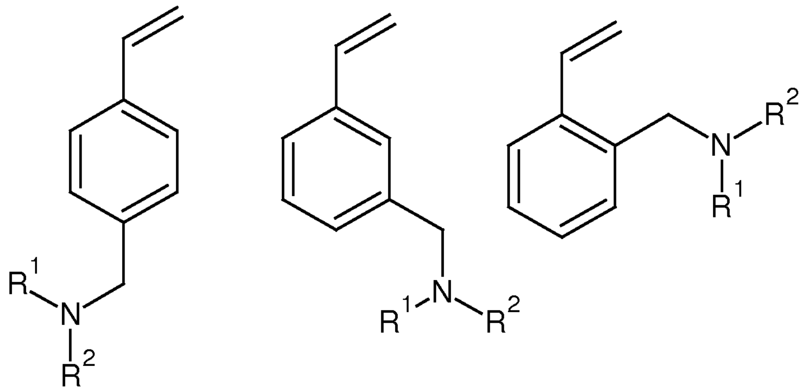

als Reaktivverdünner, wobei

$R^1$ und/oder $R^2$ unabhängig ausgewählt sind aus der Gruppe Methyl, Ethyl, n-Propyl, iso-Propyl, n-Butyl, sec-Butyl, iso-Butyl, tert.-Butyl, n-Pentyl, 3-Pentyl, iso-Pentyl, tert.-Pentyl, Cyclopentyl, Cyclohexyl,
wobei $R^1$ auch H sein kann.

Diese Monomere haben sich bei der Entwicklung der erfindungsgemäßen Werkstoffe als besonders geeignet erwiesen, antimikrobielle Werkstoffe zu erzeugen.

Ganz besonders bevorzugt ist die Verwendung der Stoffe mit $R^1$ und/oder $R^2$ ausgewählt aus Ethyl, iso-Propyl, tert.-Butyl, tert.-Pentyl,
wobei $R^1$ auch H sein kann.

In einer ganz besonders bevorzugten Ausführungsform ist $R^1$ gleich Wasserstoff und $R^2$ ist ausgewählt aus iso-Propyl, tert.-Butyl, tert.-Pentyl.

In der vorliegenden UP-Harz-Zusammensetzung sind bevorzugt je Doppelbindung in Komponente a) 0,5 bis 8 Styrolderivatmoleküle (Komponente b)) vorhanden. Anders gesagt ist das Verhältnis von Styrolderivatmolekülen b) zu Doppelbindung aus Komponente a) bevorzugt 0,5:1 bis 8:1. Die Anzahl der Styrolderivatmoleküle/Doppelbindungen in Komponente a) ist proportional zu den entsprechenden Molmengen, so dass sich das genannte Verhältnis auch mit deren Hilfe berechnen lässt.

Die Molmenge des Styrolderivats (Komponente b)) lässt sich aus der eingesetzten Menge und ihrer Molmasse gemäß

$$n_{Sty}=m_{Sty}/M_{Sty}$$

bestimmen, wobei $m_{Sty}$ die eingesetzte Menge und $M_{Sty}$ das Molgewicht des Styrolderivats sind.

Die Molmenge der Doppelbindungen in Komponente a) lässt sich aus der eingesetzten Menge und der Molmasse der Komponente a) gemäß

$$n_{Dop}=f\cdot m_{Dop}/M_{Dop}$$

bestimmen, wobei f die Anzahl an Doppelbindungen in einem Molekül der Komponente a) sowie $m_{Dop}$ die eingesetzte Menge und $M_{Dop}$ das Molgewicht der Komponente a) sind.

## Zusammensetzung

Die Zusammensetzung enthält vorzugsweise wenigstens 20 Gew.-%, besonders bevorzugt wenigstens 50 Gew.-%, ganz besonders bevorzugt wenigstens 80 Gew.-% einer Mischung der Komponenten a) und b). Im Übrigen kann die Zusammensetzung beispielsweise andere Monomere, Oligomere, Polymere, Lichtschutzmittel, Initiatoren, Additive, Pigmente, Trennmittel, Rheologieadditive, Fasern und/oder Füllstoffe enthalten.

Auf 100 Gewichtsteile einer Mischung aus den Komponenten a) und b) kommen in der Zusammensetzung vorzugsweise 0,2 bis 4 Gewichtsteile Radikalinitiator. Dieser Radikalinitiator ist vorzugsweise kein peroxidischer Radikalinitiator. Besonders bevorzugt ist der Radikalinitiator ein Photoinitiator, z.B. ein Derivat des Benzoins, des Benzils oder ein $\alpha$-Hydroxyketon oder ein $\alpha$-Aminoketon oder ein Acylphosphinoxid oder ein Bisacylphosphinoxid. Dem Fachmann ist eine Vielzahl von Photoinitiatoren bekannt. Photoinitiatoren, die C-Radikale bilden, sind bevorzugt. Besonders bevorzugt sind C-Radikalgeneratoren vom Typ der Azoinitiatoren, z.B. 2,2'-Azobis(2-methylpropionitril), auch als AIBN bezeichnet, 1,1'-Azobis(cyclohexan-1-carbonitril) oder Dimethyl-2,2'-azobis(2-methylpropionat) und sogenannte C-C-labile Verbindungen, z.B. 2,3-Dimethyl-2,3-diphenylbutan oder 3,4-Dimethyl-3,4-diphenylhexan.

Beim Radikalinitiator kann es sich auch um eine Mischung verschiedener Initiatoren handeln.

Auch ist die Härtung mit energiereicher Strahlung, z.B. Elektronenstrahlung, möglich.

Auf 100 Gewichtsteile einer Mischung aus den Komponenten a) und b) kommen in der Zusammensetzung vorzugsweise 20 bis 280 Gewichtsteile Füllstoffe.

Auf 100 Gewichtsteile einer Mischung aus den Komponenten a) und b) kommen in der Zusammensetzung vorzugsweise 10 bis 200 Gewichtsteile Glasfasern, Kohlenstofffasern, Aramidfasern, Basaltfasern, Naturfasern oder textile Vliese.

Weitere Zusatzstoffe wie beispielsweise Lichtschutzmittel, schrumpfmindernde thermoplastische Polymere, Eindickmittel, Trennmittel, Hautbildner und Wachse können je nach Verarbeitungsverfahren und Anwendung eingesetzt werden.

Die erfindungsgemäße Harzzusammensetzung, beispielsweise nach Zugabe von Additiven, Fasern und Füllstoffen, kann zur Herstellung von Sheet Molding Compounds (SMC) und Bulk Molding Compounds (BMC) sowie anderer Compounds verwendet werden.

## Verwendung der UP-Harze

In einer weiteren Ausführungsform wird die der Erfindung zu Grunde liegende Aufgabe gelöst durch Anwendung der erfindungsgemäßen UP-Harze in einem der folgenden Verarbeitungsverfahren: Beschichten, Lackieren, Gießen, Tauchen, Laminieren, Spaltimprägnieren, Schleudern, Kleben, Harzinjektion, Pressen, Spritzguss, Profilziehen, Spachteln und Wickeln.

In einer weiteren Ausführungsform wird die der Erfindung zu Grunde liegende Aufgabe gelöst durch die Verwendung der erfindungsgemäßen UP-Harze und ihrer erfindungsgemäßen Zusammensetzungen z.B. in der Möbelindustrie, in der Medizin und im Gesundheitswesen, in der medizintechnischen Industrie, in Krankenhäusern, Arztpraxen, Altenheimen und Rehabilitationszentren, in der häuslichen Alten- und Krankenpflege, in der Lebensmittel- und Fleisch erzeugenden, -verarbeitenden und -verpackenden Industrie, in der Verpackungsindustrie, in der Lagerung und Logistik, in der Dichtungsindustrie, in der Tierzucht- und Agrarindustrie und der häuslichen Tierhaltung, in der Pharmaindustrie, in der Haushaltswarenindustrie, im Apparate-, Behälter-, Rohrleitungsbaubau, in der Elektro-, Kfz- und Bauindustrie, in der Luftfahrtindustrie, in der Textilindustrie, der Hygieneartikelindustrie, in der Sanitärindustrie, in der Sport-, Spielwaren- und Freizeitartikelindustrie, im Schiffsbau, im Bootsbau, der Wassersportindustrie, der Lüftungs- und Klimatechnik, der öffentlichen, häuslichen und industriellen Wasserversorgung, der Wasseraufbereitung.

## Herstellung gehärteter Erzeugnisse

In einer weiteren Ausführungsform wird die der Erfindung zu Grunde liegende Aufgabe gelöst durch ein Verfahren zur Herstellung gehärteter Erzeugnisse, wobei man die erfindungsgemäße Zusammensetzung härtet.

Bei der Aushärtung wird die Temperatur vorteilhafterweise auf einen Bereich von 20 bis 200 °C eingestellt, mit Photoinitiatoren sind auch tiefere Temperaturen möglich.

Vorzugsweise wird das gehärtete Erzeugnis, zum Beispiel ein UP-Duromer, nach dem erfindungsgemäßen Verfahren zur Herstellung gehärteter Erzeugnisse erhalten. Das UP-Duromer besteht beispielsweise aus dem Polyester und oligomeren Brückenstrukturen, die sich aus dem Reaktivverdünner gebildet haben und die vorzugsweise überwiegend eine mittlere Kettenlänge von 1,5 bis 4 monomeren Reaktivverdünnereinheiten aufweisen.

Der E-Modul des UP-Duromers liegt vorteilhafterweise in einem Bereich von 2000 bis 4000 $N/mm^2$. Die UP-Duromere weisen vorteilhafterweise eine Dehnung in einem Bereich von 0,5 bis 6 % auf. Sie sind vorzugsweise geruchsfrei.

Das gehärtete Polyesterharz (UP-Duromer) kann faserverstärkt oder unverstärkt sein, mit Füllstoffen gefüllt oder ungefüllt sein und unabhängig davon in technischen Anwendungen der verschiedensten Art, in der Lebensmittelbranche, in Krankenhäusern und bei medizinischen Geräten, in Kühlschränken, Kühlhäusern und vielen anderen Bereichen eingesetzt werden. Das antimikrobielle Verhalten ist eine intrinsische Materialeigenschaft und wird nicht durch konventionelle, additiv zugegebene Biozide bewirkt. Damit unterscheidet sich die erfindungsgemäße ungesättigte Polyesterharz-Zusammensetzung und die daraus erhaltenen Duromere und Werkstoffe signifikant vom heutigen Stand der biozid ausgerüsteten Kunststoffe, die üblicherweise mit Nanosilber, Isothiazolinonen, chlororganischen Verbindungen, Triazinderivaten, Verbindungen des Kupfers, Zinns, Zinks und Arsens sowie anderen Wirkstoffen arbeiten. Diese konventionellen Biozide sind wegen einer (meist langsamen) Abgabe an die Umwelt, teilweise schlechter biologischer Abbaubarkeit, eines Schwermetallgehalts, einer möglichen Anreicherung in einzelnen Organismen und/oder einer Verteilung und Verbreitung über die Nahrungsmittelkette umstritten. Mit den neuen intrinsisch antimikrobiellen Polyesterharzen werden diese Nachteile zuverlässig vermieden.

**Erzeugnis mit antimikrobieller Wirkung**

In einer weiteren Ausführungsform wird die der Erfindung zu Grunde liegende Aufgabe gelöst durch ein Erzeugnis mit antimikrobieller Wirkung enthaltend das erfindungsgemäße ausgehärtete Polyesterharz.

Das erfindungsgemäße Erzeugnis besteht zu mehr als 20 Gew.-%, vorzugsweise zu mehr als 50 Gew.-% und besonders bevorzugt zu mehr als 80 Gew.-% aus den Komponenten a) und b).

Das Erzeugnis ist vorzugsweise ein Klebstoff, eine Dichtungsmasse, eine Vergussmasse, eine Beschichtung oder ein Formkörper.

In einer weiteren Ausführungsform wird die der Erfindung zu Grunde liegende Aufgabe gelöst durch die Verwendung der erfindungsgemäßen UP-Harze zur Herstellung der folgenden Produkte:

Möbel und Möbeloberflächen, Kleber, Furniere und Papierlaminate, Knöpfe, Griffe, Tasten, Schalter und Gehäuse, Platten, Fußböden, Rohre, Profile, Tanks und Behälter aller Art, insbesondere für Trinkwasser, Lebensmittel und Öle, Auskleidungen aller Art, Dachbeschichtungen, Lichtplatten, Dichtmassen, Kitte, Dübelmassen, Polymerbeton, Agglomarmor, Küchenspülen, Duschtrassen, Badewannen, Waschbecken, Klobrillen, Gartenmöbel, Gartenzäune, Fassadenplatten, Kellerfensterschächte, Fahrzeugteile, Leuchtenträger, Windkraftanlagen, Imprägnierungen, Bindemittel, Vergussmassen, Spachtelmassen und/oder Reaktionsmörtel, Beschichtungen, Lacke, Gelcoats, Topcoats, Schiffe, Boote, Freizeitartikel.

**Verfahren zur Herstellung des aminofunktionalisierten Styrolderivats**

In einer weiteren Ausführungsform wird die der Erfindung zu Grunde liegende Aufgabe gelöst durch ein Verfahren zur Herstellung eines aminofunktionalisierten Styrolderivats, wobei

a) in einem ersten Schritt eine wässrige Alkalihydroxid-Lösung mit einer Konzentration in einem Bereich von 3 bis 7 mol/l (mindestens moläquivalent zur Stoffmenge an Halogenalkyl-Styrol) bereitgestellt wird,

b) in einem zweiten Schritt zu dieser wässrigen Alkalihydroxid-Lösung ein Amin mit wenigstens einem an das Stickstoffatom gebundenen Wasserstoffatom zugefügt wird,

c) in einem dritten Schritt ein Halogenalkyl-Styrol in einer Menge von 0,2 bis 0,75 Moläquivalenten bezogen auf die Menge Amin zugefügt wird,

d) in einem vierten Schritt die entstandene Reaktionslösung nach vollständiger Zugabe des Halogenalkyl-Styrols noch über einen Zeitraum von 4 bis 120 h gerührt wird, und

e) in einem fünften Schritt das entstandene aminofunktionalisierte Styrolderivat von der übrigen Reaktionslösung abgetrennt wird.

Das Halogenalkyl-Styrol ist vorzugsweise am aromatischen Ring in ortho- und/oder meta- und/oder para-Position mit der Halogenalkyl-Gruppe funktionalisiert.

Vorzugsweise wird die wässrige Alkalihydroxid-Lösung mit einer Konzentration in einem Bereich von 4,5-5,5 mol/l bereitgestellt. Die Alkalihydroxid-Lösung ist vorzugsweise eine Natriumhydroxid-Lösung. Diese Lösung hat vorteilhafterweise eine Temperatur in einem Bereich von 20 bis 30 °C.

Das Amin fällt vorteilhafterweise unter die Formel $NHR^1R^2$, wobei

$R^1$ ausgewählt ist aus H, linearen oder verzweigten oder zyklischen Alkylresten aufweisend 1 bis 10 Kohlenstoffatome und

$R^2$ ein linearer oder verzweigter oder zyklischer Alkylrest aufweisend 1 bis 10 Kohlenstoffatome ist.

Nach vollständiger Zugabe des Amins wird die Reaktionslösung vorzugsweise auf eine Temperatur in einem Bereich von 60 bis 85 °C eingestellt.

Im Halogenalkyl-Styrol ist die Alkylgruppe vorzugsweise mit nur einem Halogenatom substituiert. Die Alkylgruppe ist vorzugsweise Methyl. Das Halogenatom ist vorzugsweise Chlor.

Das Halogenalkyl-Styrol wird vorzugsweise als Lösung in Tetrahydrofuran zugegeben. Die Konzentration der Lösung in Tetrahydrofuran liegt vorzugsweise in einem Bereich von 2 bis 3 mol/l. Vorzugsweise wird das Halogenalkyl-Styrol zu der bisherigen Reaktionslösung zugetropft und die Reaktionslösung vorzugsweise für eine Zeitdauer in einem Bereich von 4 bis 120 h gerührt. Danach findet die Abtrennung vorzugsweise durch Vakuumdestillation statt.

Ein weiterer Gegenstand der vorliegenden Erfindung ist ein Stoff ausgewählt aus

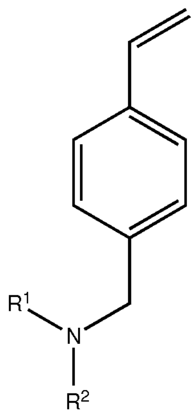
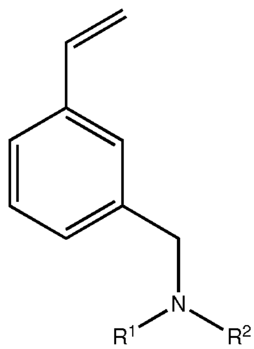
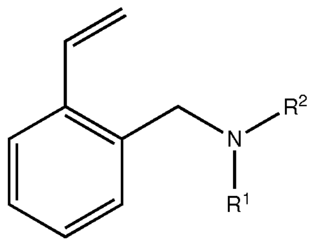

wobei $R^1$ Wasserstoff und $R^2$ tert.-Pentyl ist.

**Verwendung des aminofunktionalisierten Styrolderivats**

In einer weiteren Ausführungsform wird die der Erfindung zu Grunde liegende Aufgabe gelöst durch die Verwendung des erfindungsgemäßen aminofunktionalisierten Styrolderivats zur Herstellung von antimikrobiellen Beschichtungen oder Formkörpern.

**Ausführungsbeispiele**

Herstellung des aminofunktionalisierten Styrolderivats (allgemeine Vorschrift)

In einen 1000 ml Kolben wurden 200 ml Wasser und 42 g (1,05 mol) NaOH gegeben und nach vollständigem Lösen 1,05 mol des entsprechenden Amins zugefügt. Unter Rühren wurde der Kolben auf 60-85 °C aufgeheizt und innerhalb von etwa 75 Minuten eine Lösung aus 53,42 g (0,35 mol) Chlormethylstyrol und 150 ml THF zugetropft. Nach vollständigem Zutropfen wurde der Reaktionskolben bis zu einer Gesamtreaktionszeit von 4-120 h unter ständigem Rühren im Ölbad belassen, dabei sind die Reaktionszeit und die Reaktionstemperatur vom verwendeten Amin abhängig. Die Analytik erfolgte mittels GC-MS. Die Aufreinigung erfolgte durch Vakuumdestillation.

Nach dieser allgemeinen Vorschrift wurden aminofunktionalisierte Styrolderivate unter Einsatz folgender Amine synthetisiert: tert.-Butylamin, n-Propylamin, iso-Propylamin, n-Butylamin, sec-Butylamin, iso-Butylamin, n-Pentylamin, 3-Pentylamin, iso-Pentylamin, tert.-Pentylamin, Cyclopentylamin, Cyclohexylamin, Diethylamin, Diisopropylamin.

Das mit tert.-Butylamin erhaltene tert.-Butylaminomethylstyrol erhielt die Abkürzung TBAMS. Es wurde bei einer Reaktionstemperatur von 70 °C und einer Nachrührzeit von 24 h mit einem Umsatz von > 98% und einer Selektivität von > 98% hergestellt. Bei der Vakuumdestillation war der Siedepunkt von TBAMS 115 °C bei 6 mbar.

**Beispiel 1**

Kondensation von UP 1 ($FS_{1,0}$ $DEG_{0,5}$ $NPG_{0,5}$)

Zur Herstellung des Polyesters wurden 719,63 g Fumarsäure, 329,24 g Diethylenglykol und 322,92 g Neopentylglykol in den 2 l Vierhalskolben einer Kondensationsapparatur eingewogen und nach Zugabe von 100 ppm Hydrochinon und 220 ml Wasser unter ständigem Rühren und kontinuierlichem Stickstoffstrom (5 l/h) auf 80 °C erwärmt. Anschließend wurde die Reaktionstemperatur mit einer Heizrate von etwa 10 K/10min auf 210 °C erhöht. Unter ständiger Wasserabscheidung wurde die Reaktionstemperatur von 210 °C für 2,5 h gehalten. Danach wurde der Polyester auf 115 °C abgekühlt und in Braunglasflaschen abgefüllt.

Die durch Titration ermittelte Säurezahl betrug 28 mg KOH/g UP. Die mittels I.C.I.-Kegel-Platte-Viskosimeter bestimmte Schmelzviskosität betrug 780 mPas (150 °C, 10000 $s^{-1}$).

1A. Harzherstellung und Härtung (2,5 mol TBAMS je mol UP 1-Doppelbindung)

Zur Harzherstellung wurden der ungesättigte Polyester (56,2 g; 28,1 Gew.-%) und der aminofunktionalisierte Reaktivverdünner TBAMS (tert.-Butylaminomethylstyrol) (143,8 g; 71,9 Gew.-%) zum Lösen 14 Tage auf einen Rollbock gelegt und nach vollständigem Lösen mit 2 Gew.-% des Azoinitiators V601 [Dimethyl 2,2'-azobis(2-methylpropionat)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

Der Polyesteranteil entspricht Komponente a) und beträgt 56,2 g (28,1 Gew.-%). Die konstitutive Repetiereinheit des Polyesters ist wie folgt aufgebaut:

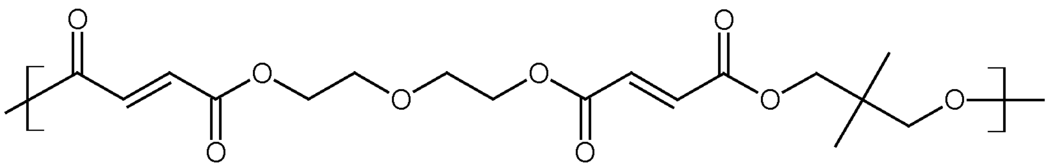

Die Molmasse der Repetiereinheit beträgt 370,35 g/mol und enthält 2 reaktive Fumarsäure-Doppelbindungen.

$$\text{Somit ergibt sich } n_{Dop}=f\cdot m_{Dop}/M_{Dop}=2\cdot 56{,}2\text{ g}/370{,}35\text{ g/mol}=0{,}303\text{ mol}$$

Der Styrolderivatanteil (tert.Butylaminomethylstyrol, TBAMS) entspricht Komponente b) und beträgt 143,8 g (71,9 Gew. %). Das Molgewicht beträgt 189,3 g/mol.

$$\text{Somit ergibt sich } n_{Sty}=m_{Sty}/M_{Sty}=143{,}8\text{g}/189{,}3\text{ g/mol}=0{,}760\text{ mol}$$

Das Verhältnis von Styrolderivat zu Doppelbindung beträgt:

$$n_{Sty}: n_{Dop}= 0{,}760\text{ mol}:0{,}303\text{ mol}= 2{,}51 : 1$$

1 B. Harzherstellung und Härtung (2,25 mol TBAMS je mol UP 1-Doppelbindung)

Zur Harzherstellung wurden der ungesättigte Polyester (45,5 g; 30,3 Gew.-%) und der aminofunktionalisierte Reaktivverdünner TBAMS (tert.-Butylaminomethylstyrol) (104,6 g; 69,7 Gew.-%) zum Lösen 14 Tage auf einen Rollbock gelegt und nach vollständigem Lösen mit 2 Gew.-% des Azoinitiators V601 [Dimethyl 2,2'-azobis(2-methylpropionat)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

In diesem Ausführungsbeispiel beträgt das Verhältnis von Styrolderivat zu Doppelbindung in Komponente a) 2,25 : 1.

1C. Harzherstellung und Härtung (2,0 mol TBAMS je mol UP 1-Doppelbindung)

Zur Harzherstellung wurden der ungesättigte Polyester (46,1 g; 32,9 Gew.-%) und der aminofunktionalisierte Reaktivverdünner TBAMS (tert.-Butylaminomethylstyrol) (93,9 g; 67,1 Gew.-%) zum Lösen 14 Tage auf einen Rollbock gelegt und nach vollständigem Lösen mit 2 Gew.-% des Azoinitiators V601 [Dimethyl 2,2'-azobis(2-methylpropionat)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

In diesem Ausführungsbeispiel beträgt das Verhältnis von Styrolderivat zu Doppelbindung in Komponente a) 2,00 : 1.

**Beispiel 2**

Kondensation von UP 2 ($FS_{0,5}THPS_{0,5}NPG_{1,0}$)

Zur Herstellung des Polyesters wurden 348,21 g Fumarsäure, 456,45 g Tetrahydrophthalsäureanhydrid und 643,65 g Neopentylglykol in den 2 l Vierhalskolben einer Kondensationsapparatur eingewogen und nach Zugabe von 200 ppm Hydrochinon, 400 ppm Fascat 4100 (n-Butylzinnsäure) und 170 g Wasser unter ständigem Rühren und kontinuierlichem Stickstoffstrom (5 l/h) auf 140 °C erwärmt. Anschließend wurde die Reaktionstemperatur mit einer Heizrate von etwa 10 K/30min auf 200 °C erhöht. Unter ständiger Wasserabscheidung wurde die Reaktionstemperatur für 4 h gehalten, anschließend auf 210 °C erhöht und weitere 2 h gehalten. Nach Abkühlen auf 120 °C wurde der Polyester in Braunglasflaschen abgefüllt.

Die durch Titration ermittelte Säurezahl betrug 29,8 mg KOH/g UP. Die mittels I.C.I.-Kegel-Platte-Viskosimeter bestimmte Schmelzviskosität betrug 770 mPas (150 °C, 10000 $s^{-1}$).

2A. Harzherstellung und Härtung (2,5 mol TBAMS je mol UP 2-Doppelbindung)

Zur Harzherstellung wurden der ungesättigte Polyester UP 2 (47,1 Gew. %) und der aminofunktionalisierte Reaktivverdünner TBAMS (tert.-Butylaminomethylstyrol) (52,9 Gew.-%), zum Lösen 14 Tage auf einen Rollbock gelegt und nach vollständigem Lösen mit 2 Gew.-% des Azoinitiators V601 [Dimethyl 2,2'-azobis(2-methylpropionate)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

2B. Harzherstellung und Härtung (2,25 mol TBAMS je mol UP 2-Doppelbindung)

Zur Harzherstellung wurden der ungesättigte Polyester UP 2 (49,8 Gew.-%) und der aminofunktionalisierte Reaktivverdünner TBAMS (tert.-Butylaminomethylstyrol) (50,2 Gew.-%), zum Lösen 14 Tage auf einen Rollbock gelegt und nach vollständigem Lösen mit 2 Gew.-% des Azoinitiators V601 [Dimethyl 2,2'-azobis(2-methylpropionate)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

2C. Harzherstellung und Härtung (2,0 mol TBAMS je mol UP 2-Doppelbindung)

Zur Harzherstellung wurden der ungesättigte Polyester UP 2 (52,7 Gew.-%) und der aminofunktionalisierte Reaktivverdünner TBAMS (tert.-Butylaminomethylstyrol) (47,3 Gew.-%), zum Lösen 14 Tage auf einen Rollbock gelegt und nach vollständigem Lösen mit 2 Gew.-% des Azoinitiators V601 [Dimethyl 2,2'-azobis(2-methylpropionate)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

**Beispiel 3**

Kondensation von UP-3 ($MSA_{1,0}DEG_{0,5}NPG_{0,5}$)

Zur Herstellung des Polyesters wurden 637,40 g Maleinsäureanhydrid, 345,18 g Diethylenglykol und 338,54 g Neopentylglykol in den 2 l Vierhalskolben einer Kondensationsapparatur eingewogen und nach Zugabe von 100 ppm Hydrochinon unter ständigem Rühren und kontinuierlichem Stickstoffstrom (5 l/h) auf 80 °C erwärmt. Anschließend wurde die Reaktionstemperatur mit einer Heizrate von etwa 10 K/10min auf 200 °C erhöht. Unter ständiger Wasserabscheidung wurde die Reaktionstemperatur für 3 h gehalten. Anschließend wurde der Polyester durch Ausschalten der Temperierung auf 115 °C abgekühlt und in Braunglasflaschen abgefüllt.

Die durch Titration ermittelte Säurezahl betrug 29,4 mg KOH/g UP. Die mittels I.C.I.-Kegel-Platte-Viskosimeter bestimmte Schmelzviskosität betrug 220 mPas (150 °C, 10000 $s^{-1}$).

3A. Harzherstellung und Härtung (2,5 mol TBAMS je mol UP 3-Doppelbindung)

Zur Harzherstellung wurden der ungesättigte Polyester UP 3 (28,1 Gew.-%) und der aminofunktionalisierte Reaktivverdünner TBAMS (tert.-Butylaminomethylstyrol) (71,9 Gew.-%), zum Lösen 14 Tage auf einen Rollbock gelegt und nach vollständigem Lösen mit 2 Gew.-% des Azoinitiators V601 [Dimethyl 2,2'-azobis(2-methylpropionate)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

3B. Harzherstellung und Härtung (2,0 mol TBAMS je mol UP 3-Doppelbindung)

Zur Harzherstellung wurden der ungesättigte Polyester UP 3 (32,9 Gew.-%) und der aminofunktionalisierte Reaktivverdünner TBAMS (tert.-Butylaminomethylstyrol) (67,1 Gew.-%), zum Lösen 14 Tage auf einen Rollbock gelegt und nach vollständigem Lösen mit 2 Gew.-% des Azoinitiators V601 [Dimethyl 2,2'-azobis(2-methylpropionate)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

**Beispiel 4**

Aminofunktionalisierung von UP 3 ($MSA_{1,0}DEG_{0,5}NPG_{0,5}$)

237,10 g des ungesättigten Polyesters UP-3 wurden in einen 1 L-Kolben mit Rückflusskühler gefüllt und auf 65 °C erwärmt. Unter Rühren wurden innerhalb von 20 Minuten 11,7g (entspricht 12,5 mol% der Fumar- und Maleinestereinheiten des UP-3) tert.-Butylamin zugetropft und die Temperatur dann schrittweise auf 140 °C erhöht und eine Stunde gehalten. Anschließend wurde der ungesättigte Polyester auf 115 °C abgekühlt und in Braunglasflaschen abgefüllt.

Harzherstellung und Härtung

Zur Harzherstellung wurden 50 Gew.-% des funktionalisierten ungesättigten Polyesters UP-3 und 50 Gew.-% des aminofunktionalisierten Reaktivverdünners TBAMS (tert.-Butylaminomethylstyrol) in eine Braunglasflasche gefüllt und zum Lösen 14 Tage auf einen Rollbock gelegt. Nach vollständigem Lösen wurde das Harz mit 2 Gew.-% des

Azoinitiators V601 [Dimethyl 2,2'-azobis(2-methylpropionat)] der Fa. Wako versetzt. Anschließend wurden jeweils etwa 8 g des transparenten homogenen Harzes auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

**Beispiel 5**

Kondensation von UP-5 ($FS_{1,0}DEG_{0,306}NPG_{0,51}TBBHEA_{0,204}$)

Zur Herstellung des Polyesters wurden 928,56 g Fumarsäure 259,73 g Diethylenglykol, 424,93 g Neopentylglykol, 263,14 g tert.-Butyl-bis-hydroxyethylamin (TBBHEA) und 100 g Wasser in den 2 l Vierhalskolben einer Kondensationsapparatur eingewogen und nach Zugabe von 200 ppm Hydrochinon unter ständigem Rühren und kontinuierlichem Stickstoffstrom (20 l/h) innerhalb von 2 h auf 140 °C erwärmt und 55 min. unter beginnender Wasserabscheidung bei 140 °C gehalten. Anschließend wurde die Reaktionstemperatur mit einer Heizrate von etwa 15 K/10min auf 170 °C erhöht, etwa 30 min bei 170 °C gehalten und anschließend auf RT abgekühlt. Am nächsten Tag wurde der Reaktionsansatz innerhalb von etwa 210 min gleichmäßig auf 185 °C erwärmt. Anschließend wurde die Temperatur innerhalb von 20 min. auf 190 °C erhöht und unter weiterer Wasserabscheidung für 90 min. bei 190 °C gehalten. Daran gefolgt wurde der Polyester durch Ausschalten der Temperierung auf 115 °C abgekühlt und in Braunglasflaschen abgefüllt.

Die durch Titration ermittelte Säurezahl betrug 34,7 mg KOH/g UP. Die mittels I.C.I.-Kegel-Platte-Viskosimeter bestimmte Schmelzviskosität betrug 920 mPas (150 °C, 1250 $s^{-1}$).

Harzherstellung und Härtung

Zur Harzherstellung wurden 51,72 g (40 Gew.-%) des UP-5, 77,58 g (60 Gew.-%) des amino-funktionalisierten Reaktivverdünners TBAMS (tert.-Butylaminomethylstyrol) und 200 ml Aceton in einen Einhalskolben gegeben und bis zum vollständigen Lösen des ungesättigten Polyesters gerührt. Daran gefolgt wurde das Aceton mittels eines Rotationsverdampfers im Vakuum abgetrennt. Nach Abtrennung des Acetons wurden 2 Gew.-% des Azoinitiators V601 [Dimethyl 2,2'-azobis(2-methylpropionat)] der Fa. Wako hinzugefügt. Nach Homogenisierung der Harz-Initiator-Mischung wurden jeweils etwa 8 g auf verschiedene Glaspetrischalen gegeben und unter Stickstoffatmosphäre bei 70 °C für 2 h, bei 80 °C für 2 h und bei 90 °C für 2 h gehärtet.

Das erhaltene, voll ausgehärtete UP-Duromer war klebfrei und hart. Das UP-Duromer war praktisch geruchsfrei und zeigte exzellente antimikrobielle Eigenschaften.

**Vorgehen zur Bestimmung des dynamisch-mechanischen Verhaltens und der Glasübergangstemperatur**

Zur Bestimmung der Netzwerk-$T_G$ wurden aus den Harzen der Beispiele 1C, 2A, 2C, 3B und 4 glasfaserverstärkte Duromer-Probekörper hergestellt und mittels dynamischmechanischer Analyse (DMA) charakterisiert.

Die verwendete DMA 242 der Fa. Netzsch ermöglicht die Bestimmung des Speicher- und Verlustmoduls, sowie des Verlustfaktors einer Probe als Funktion der Temperatur und Frequenz einer aufgebrachten sinusförmigen Schwingungsbelastung.

Zur Herstellung der benötigten Probekörper wurden die in den Ausführungsbeispielen hergestellten, mit 2 Gew.-% Initiator (V601 Fa. Wako) versetzten Harze verwendet.

Hierzu wurden drei 15x15 cm Lagen Saertex®-Glasfasergelege (biaxial 0°/90°/ Typ: S14EB540-00620-T1300-487000) mit dem jeweiligen Harz imprägniert, in eine verschraubbare, mit Mylar®-Folie ausgekleidete 150x150x5 mm Plattenform gelegt und durch Andrücken mit einem Spatel möglichst von Luftblasen befreit. Anschließend wurde die Kavität der Form mit weiterem Harz vollständig gefüllt, mit Mylar®-Folie abgedeckt und mit der oberen Werkzeugplatte durch Verschraubung verschlossen. Die Härtung erfolgte für jeweils 2 h bei 70, 80 und 90 °C in einem Trockenschrank.

Nach Abkühlen wurden die GFK-Platten mit einer Tischkreissäge zugeschnitten und ggf. mit einem Schleifband auf die erforderliche Probengröße gebracht.

Parameter und Messeinstellungen bei den durchgeführten DMA-Analysen:

Probenabmessungen: 50 x 10 x 5 mm

Deformationsmodus: Zweiarmige Biegung

Amplitude: 30 μm

Dynamische Kraft: 7,55 N

Statische Kraft: 4 N

Temperaturbereich: 20 - 160 °C

Heizrate: 2 K/min

Frequenz: 1 Hz / 10 Hz

Atmosphäre: $N_2$

Flussrate $N_2$: 5 ml/min

**Vorgehen bei den antimikrobiellen Untersuchungen**

Die angewendete Methode basierte auf dem Japanischen Standard JIS Z 2801:2000. Testmikroorganismus für die Versuche war der pathogene Keim Staphylococcus aureus. Es wurde ein Standardkeim (ATCC 6538) verwendet - nicht ein multiresistenter.

Bei jedem Testkeim (hier Staphylococcus aureus) stellte man unter den Bedingungen der Herstellung der Ausgangslösung oder Ausgangssuspension einen Mikroorganismus-spezifischen Keimgehalt ein. Bei Staphylococcus aureus betrug dieser Wert 108 Keime pro ml (siehe auch Ausführungen weiter unten).

Die Bestimmung der antimikrobiellen Aktivität erfolgte durch den Vergleich des Wachstums von Staphylococcus aureus auf Referenzoberflächen und auf den Probenmaterialien.

Als Referenzmaterial dienten leere Petrischalen. Die Proben bestanden aus Petrischalen, in die jeweils eine dünne Schicht einer Polymerprobe gegossen war. Für jede Versuchsreihe wurden drei Referenzplatten zur Bestimmung des Anfangskeimgehaltes (separater Versuch unabhängig von der Untersuchung des antimikrobiellen Verhaltens) sowie drei Referenzplatten und drei Probenplatten zur Bestimmung des Oberflächenkeimgehaltes nach der Inkubation herangezogen.

Alle Platten wurden mit 400 µl Staphylococcus aureus Impfsuspension beimpft, die auf einen Keimgehalt von 4,0 - 10 * $10^5$ KbE/ml eingestellt war.

Die aufgebrachte Impfsuspension wurde mit einer sterilen PP-Folie abgedeckt, um Verdunstung zu vermeiden. Unmittelbar nach dem Beimpfen wurden die drei Probenplatten und drei Referenzplatten in einen Inkubationsschrank überführt und bei 35 °C und 90 % Luftfeuchte für 2 h bzw. 24 h gelagert.

Zur Bestimmung des Keimgehaltes in der Beimpfungslösung (Anfangskeimgehalt) wurden je drei Referenzplatten direkt nach dem Beimpfen, unter Zugabe von 10 ml SCDLP-Bouillon (Soja-Casein-Pepton-Bouillon mit Lecithin und Polyoxyethylenmedium) in die Petrischale, ausgewaschen. Die Folie wurde mit einer sterilen Pinzette gewendet und mehrmals mit einer 1 ml-Pipette über- beziehungsweise unterspült. Die Petrischale wurde in Form einer Acht geschwenkt, bevor 1 ml der Auswaschlösung in die erste Verdünnungsstufe pipettiert wurde. Nach Anlegen einer Verdünnungsreihe wurde der Lebendkeimgehalt mittels Drop-Plate-Verfahren bestimmt. Beim Drop-Plate-Verfahren wurden - im Doppelansatz - auf einer Plate-Count-(PC)-Agar-Platte in jeden Sektor der jeweiligen Verdünnungsstufe 5 Tropfen je 10 µl aufgebracht. Die Bebrütung der Platten erfolgte für 2 h bzw. 24 h bei 37 °C.

Das Auswaschen und die Bestimmung der Lebendkeimzahl auf den Referenz- und Probenplatten nach der Inkubation erfolgten in gleicher Art und Weise wie die Bestimmung des Anfangskeimgehaltes. Bei den Probenplatten wurde zusätzlich zur Erhöhung der Nachweisgrenze der Keimgehalt der direkten Auswaschlösung im Plattengussverfahren bestimmt. Hierzu wurde, ebenfalls im Doppelansatz, je 1 ml der Lösung in eine leere Petrischale gegeben und mit flüssigem, auf 45 °C temperierten PC-Agar übergossen. Durch Schwenken in Form einer Acht wurden die Bakterien im Agar verteilt. Die Bebrütung der Platten erfolgte für 48 h bei 37 °C.

Nach der Inkubation wurden die Kolonien in der Petrischale gezählt. Dabei wurde unterstellt, dass aus jedem Keim eine sichtbare Kolonie geworden war. Die Kolonien waren nach der Bebrütung mit bloßem Auge sichtbar - zur Hilfe konnte ein Lichttisch herangezogen werden, um die Keime besser zu erkennen.

Anhand des Volumens der Beimpfungslösung und der verwendeten Verdünnungsverhältnisse konnte auf die Lebendkeimzahl der Mikroorganismen pro Volumeneinheit (d.h. pro ml) Beimpfungslösung rückgeschlossen werden. Die Berechnung erfolgte anhand des gewogenen arithmetischen Mittels mit folgender Formel:

$$\bar{c} = \frac{\sum c}{n_1 \cdot 1 + n_2 \cdot 0{,}1} \cdot d$$

Mit

$\bar{c}$ gewogenes arithmetisches Mittel

$\sum c$ Summe der Kolonien aller Petrischalen oder Sektoren, die zur Berechnung herangezogen werden

$n_1$ Anzahl der Petrischalen oder Sektoren der niedrigsten auswertbaren Verdünnungsstufe

$n_2$ Anzahl der Petrischalen oder Sektoren der nächst höheren Verdünnungsstufe

$d$ Faktor der niedrigsten ausgewerteten Verdünnungsstufe

Beim Plattengussverfahren waren Petrischalen mit bis zu 300 KbE (Kolonie bildende Einheiten) zählbar. Beim Drop-Plate-Verfahren waren nur Platten bis 150 KbE pro Sektor auswertbar.

Bei der Bestimmung der Lebendkeimzahl pro ml musste der Verdünnungsfaktor *F*1 beachtet werden. Dieser ergab sich aus der Summe des Volumens der SCDLP-Bouillon und dem Volumen der Bakteriensuspension auf der beimpften Platte dividiert durch das Volumen der Bakteriensuspension auf dieser beimpften Platte.

$$F_1 = \frac{10\ ml + 0{,}4\ ml}{0{,}4\ ml} = 26$$

$F_1$ Verdünnungsfaktor der SCDLP-Bouillon

Für die Gesamtkeimzahl auf den beimpften Proben- beziehungsweise Referenzplatten im Plattengussverfahren ergab sich somit folgende Formel:

$$KbE = \frac{\sum c}{n_1 \cdot 1 + n_2 \cdot 0{,}1} \cdot d \cdot F_1$$

Beim Drop-Plate-Verfahren war noch ein weiterer Verdünnungsfaktor relevant, da ein Viertel einer Platte nur mit 50 µl, also 0,05 ml, beimpft wurde. Um auf den Keimgehalt pro ml zu schließen, mussten 0,05 ml auf 1 ml umgerechnet werden, indem man mit 20 multiplizierte.

$$F_2 = 26 \cdot 20$$

$F_2$ Verdünnungsfaktor, um die KbE beim Drop-Plate-Verfahren pro ml zu erhalten.

Entsprechend wurde die Gesamtkeimzahl der beimpften Proben- beziehungsweise der Referenzplatten für das Drop-Plate-Verfahren unter Berücksichtigung aller Verdünnungsfaktoren nach folgender Formel berechnet:

$$KbE = \frac{\sum c}{n_1 \cdot 1 + n_2 \cdot 0{,}1} \cdot d \cdot F_2$$

Für die Berechnung der antimikrobiellen Aktivität wurden in jeder Versuchsreihe die Einzelergebnisse der Lebendkeimzahl für die Platten als einfaches arithmetisches Mittel zusammengefasst und daraus die $Log_{10}$-Reduktion zwischen Proben und Referenzplatten ermittelt.

Die Berechnung erfolgte mittels nachstehender Formel:

$$\log_{10} - \mathit{Reduktion} = \log_{10}(KG)_{\mathit{Ref}(x)} - \log_{10}(KG)_{\mathit{Pr}(x)}$$

wobei

$(KG)_{Ref(x)}$ KbE auf den Referenzplatten zum Zeitpunkt x und

$(KG)_{Pr(x)}$ KbE auf den Probenplatten zum Zeitpunkt x ist.

Laut JIS Z 2801:2000 ist eine antimikrobielle Aktivität bei einer Log-Reduktion von mindestens 2,0 nach 24 Stunden Einwirkzeit gegeben.

Waren auf den Agar-Platten der Proben bei der niedrigsten Verdünnungsstufe im Plattengussverfahren keine auszählbaren Kolonien vorhanden, wurde das Ergebnis mit <10 KbE/ml angegeben entsprechend der Vorgabe im Teststandard.

Ergebnisse der dynamisch-mechanischen und antimikrobiellen Untersuchungen

| Beispiel | Zusammensetzung UP | Massenanteil TBAMS | $T_G$ bei 1HZ | $T_G$ bei 10HZ | Anfangskeimgehalt (log KbE/ml) | Referenzkeimgehalt nach 2h (log KbE/ml) | Oberflächenkeimgehalt Probe nach 2h (log KbE/ml) | Log-Reduktion nach 2h | Referenzkeimgehalt nach 24h (log KbE/ml) | Oberflächenkeimgehalt Probe nach 24h (log KbE/ml) | Log-Reduktion nach 24h |
|---|---|---|---|---|---|---|---|---|---|---|---|
| 1A | $FS_{1,0}DEG_{0,5}NPG_{0,5}$ | 0,72 | --- | --- | 5,7 | 5,5 | 3,3 | 2,2 | -- | --- | --- |
| 1B | $FS_{1,0}DEG_{0,5}NPG_{0,5}$ | 0,70 | --- | --- | 5,7 | 5,5 | 3,6 | 1,9 | --- | --- | --- |
| 1C | $FS_{1,0}DEG_{0,5}NPG_{0,5}$ | 0,67 | 132,4 | 142,6 | 5,7 | 5,5 | 3,0 | 2,5 | --- | --- | --- |
| 2A | $FS_{0,5}THPSA_{0,5}NPG_{1,0}$ | 0,53 | 98,1 | 106,7 | 5,3 | --- | --- | --- | 8,2 | 1,0 | 7,2 |
| 2B | $FS_{0,5}THPSA_{0,5}NPG_{1,0}$ | 0,50 | --- | --- | 5,3 | --- | --- | --- | 8,2 | 1,0 | 7,2 |
| 2C | $FS_{0,5}THPSA_{0,5}NPG_{1,0}$ | 0,47 | 95,8 | 103,3 | 5,3 | --- | --- | --- | 8,2 | 1,0 | 7,2 |
| 3A | $MSA_{1,0}DEG_{0,5}NPG_{0,5}$ | 0,72 | --- | --- | 5,6 | --- | --- | --- | 7,5 | 1,0 | 6,5 |
| 3B | $MSA_{1,0}DEG_{0,5}NPG_{0,5}$ | 0,67 | 110,7 | 118,6 | 5,7 | --- | --- | --- | 7,8 | 1,0 | 6,8 |
| 4 | $MSA_{1,0}DEG_{0,5}NPG_{0,5}$+ TBA | 0,50 | 119,7 | 128,4 | 5,7 | --- | --- | --- | 7,8 | 1,0 | 6,8 |
| 5 | $FS_{1,0}DEG_{0,3}NPG_{0,5}TBBHEA_{0,2}$ | 0,60 | 119,3 | 128,9 | 5,7 | --- | --- | --- | 7,8 | 1,0 | 6,8 |

Bemerkungen:

1. Bei allen Produkten zeigt sich eine ausgeprägte antimikrobielle Wirksamkeit.

2. Für einige Proben, z.B. 2A, 2B, 2C, ergeben sich identische Werte für die log Reduktion nach 24 h. Dies ergibt sich aus der Tatsache, dass die drei Messungen parallel erfolgten, so dass der gleiche Referenzkeimgehalt zum Ansatz kam. Da die Restkeimgehalte der Proben nach 24 h jeweils unter der Nachweisgrenze des Verfahrens lagen, wurden sie gemäß Norm mit log 1 angegeben, sodass sich für die log-Reduktion identische Werte ergaben.

## Patentansprüche

**1.** UP-Harz-Zusammensetzung zur Herstellung von Erzeugnissen mit antimikrobieller Wirkung enthaltend

a) einen ungesättigten Polyester aus einerseits Dicarbonsäure und/oder Anhydrid und andererseits Diol im molaren Verhältnis 1,25:1 bis 0,75:1, wobei die Dicarbonsäure und/oder das Anhydrid wenigstens teilweise mit einer radikalisch reaktiven Doppelbindung funktionalisiert sind, und
b) Styrolderivat als Reaktivverdünner,
wobei bevorzugt je Doppelbindung in Komponente a) 0,5 bis 8 Styrolderivatmoleküle in der Zusammensetzung vorhanden sind, und
wobei wenigstens eine Entität ausgewählt aus Styrolderivat, Dicarbonsäure und/oder Diol aminofunktionalisiert ist,
wobei die Aminofunktionaliät unabhängig von der Entität unter die Formel

$$-(CH_2)_q-NH_pR^1R^2A_p$$

fällt, wobei

q entweder 0, 1 oder 2 ist, wobei $q \neq 0$ ist, sofern die Funktionalität an einen Aromaten gebunden ist,
p 0 oder 1 ist,
$R^1$ ausgewählt ist aus H, linearen oder verzweigten oder zyklischen Alkylresten aufweisend 1 bis 10 Kohlenstoffatome,
$R^2$ ein linearer oder verzweigter oder zyklischer Alkylrest aufweisend 1 bis 10 Kohlenstoffatome ist,
A das Anion einer Säure ist und
der Amin-Stickstoff N der obenstehenden Formel neutral (p = 0) oder positiv (p = 1) geladen ist.

**2.** Zusammensetzung gemäß Anspruch 1, **dadurch gekennzeichnet, dass** das Styrolderivat aminofunktionalisiert ist.

**3.** Zusammensetzung gemäß den Ansprüchen 1 oder 2, **dadurch gekennzeichnet, dass** das aminofunktionalisierte Styrolderivat einer dieser Stoffe ist

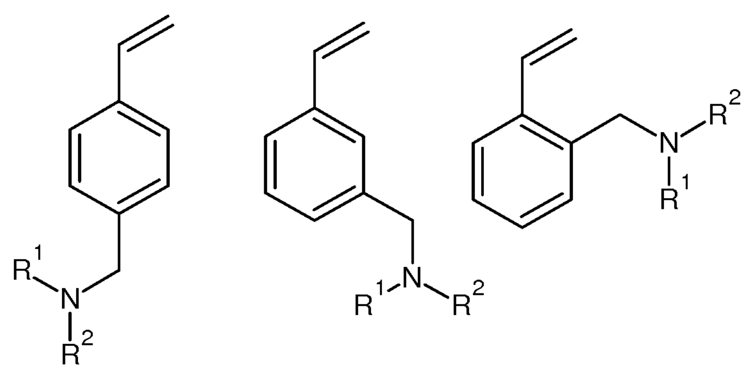

wobei $R^1$ Wasserstoff ist und
wobei $R^2$ ausgewählt ist aus der Gruppe iso-Propyl, tert.-Butyl, tert.-Pentyl.

**4.** Zusammensetzung gemäß einem der Ansprüche 1 bis 3, **dadurch gekennzeichnet, dass** sie wenigstens 20 Gew.-%, insbesondere wenigstens 80 Gew.-% einer Mischung der Komponenten a) und b) enthält.

**5.** Zusammensetzung gemäß einem der Ansprüche 1 bis 4, wobei je Doppelbindung in Komponente a) 1,5 bis 4 Styrolderivatmoleküle in der Zusammensetzung vorhanden sind.

**6.** Verwendung der Zusammensetzung gemäß einem der Ansprüche 1 bis 5 in einem der folgenden Verarbeitungsverfahren: Beschichten, Lackieren, Gießen, Tauchen, Laminieren, Spaltimprägnieren, Schleudern, Kleben, Harzinjektion, Pressverfahren, Spritzguss, Profilziehen, Spachteln und Wickeln.

**7.** Verfahren zur Herstellung gehärteter Erzeugnisse, wobei man eine Zusammensetzung gemäß einem der Ansprüche 1 bis 5 härtet.

**8.** Erzeugnis mit antimikrobieller Wirkung enthaltend eine gehärtete Zusammensetzung gemäß Anspruch 7, wobei das Erzeugnis zu mehr als 20 Gew.% aus den Komponenten a) und b) besteht.

**9.** Verwendung des Erzeugnisses nach Anspruch 8 zur Herstellung der folgenden Produkte: Möbel und Möbeloberflächen, Kleber, Furniere und Papierlaminate, Knöpfe, Griffe, Tasten, Schalter und Gehäuse, Platten, Fußböden, Rohre, Profile, Tanks und Behälter, insbesondere für Trinkwasser, Lebensmittel und Öle, Auskleidungen, Dachbeschichtungen, Lichtplatten, Dichtmassen, Kitte, Dübelmassen, Polymerbeton, Agglomarmor, Küchenspülen, Duschtrassen, Badewannen, Waschbecken, Klobrillen, Gartenmöbel, Gartenzäune, Fassadenplatten, Kellerfensterschächte, Fahrzeugteile, Leuchtenträger, Windkraftanlagen, Imprägnierungen, Bindemittel, Vergussmassen, Spachtelmassen und/oder Reaktionsmörtel, Beschichtungen, Lacke, Gelcoats, Topcoats, Schiffe, Boote, Freizeitartikel.

**10.** Verfahren zur Herstellung eines aminofunktionalisierten Styrolderivats, wobei die Aminofunktionaliät unter die Formel

$$-(CH_2)_q-NH_pR^1R^2A_p$$

fällt, wobei
q entweder 0, 1 oder 2 ist, wobei $q \neq 0$ ist, sofern die Funktionalität an einen Aromaten gebunden ist,
p 0 oder 1 ist,
$R^1$ ausgewählt ist aus H, linearen oder verzweigten oder zyklischen Alkylresten aufweisend 1 bis 10 Kohlenstoffatome,
$R^2$ ein linearer oder verzweigter oder zyklischer Alkylrest aufweisend 1 bis 10 Kohlenstoffatome ist,
A das Anion einer Säure ist und
der Amin-Stickstoff N der obenstehenden Formel neutral (p = 0) oder positiv (p = 1) geladen ist, und

wobei

a) in einem ersten Schritt eine wässrige Alkalihydroxid-Lösung mit einer Konzentration in einem Bereich 3 bis 7 mol/l bereitgestellt wird,
b) in einem zweiten Schritt zu dieser wässrigen Alkalihydroxid-Lösung eine halbmoläquivalente bis moläquivalente Menge an einem Amin mit wenigstens einem an das Stickstoffatom gebundenen Wasserstoffatom zugefügt wird,
c) in einem dritten Schritt ein Halogenalkyl-Styrol in einer Menge von 0,2 bis 0,5 Moläquivalenten bezogen auf die Menge Alkalihydroxid zugefügt wird,
d) in einem vierten Schritt die entstandene Reaktionslösung nach vollständiger Zugabe des Halogenalkyl-Styrol noch über einen Zeitraum in einem Bereich von 2 bis 48 h gerührt wird, und
e) in einem fünften Schritt das entstandene aminofunktionalisierte Styrolderivat von der übrigen Reaktionslösung abgetrennt wird.

**11.** Verwendung eines oder einer Mischung der drei Stoffe ausgewählt aus

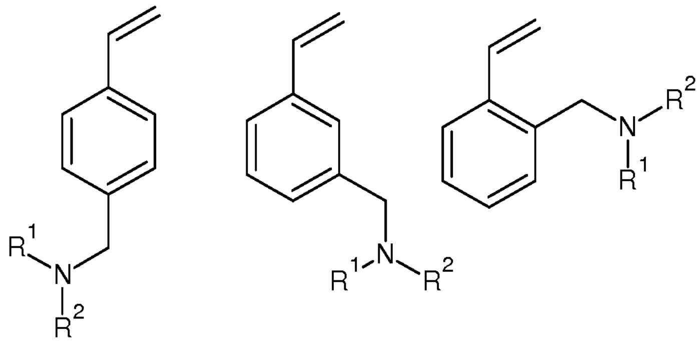

als Reaktivverdünner in Harzanwendungen,
wobei $R^1$ und/oder $R^2$ unabhängig ausgewählt sind aus der Gruppe Methyl, Ethyl, n-Propyl, iso-Propyl, n-Butyl, sec-Butyl, iso-Butyl, tert.-Butyl, n-Pentyl, 3-Pentyl, iso-Pentyl, tert.-Pentyl, Cyclopentyl, Cyclohexyl,
wobei $R^1$ auch H sein kann.

**12.** Stoff ausgewählt aus

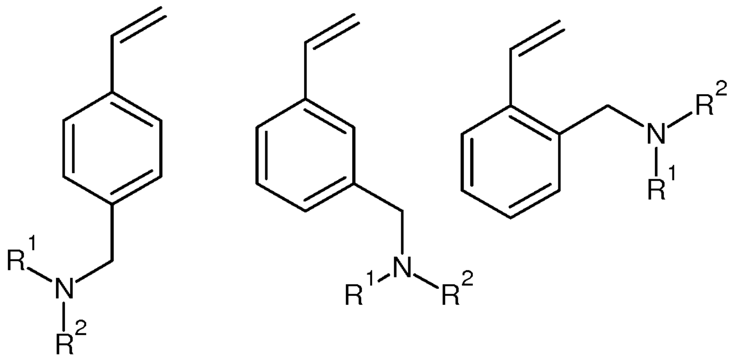

wobei $R^1$ Wasserstoff und $R^2$ tert.-Pentyl ist.

**13.** Verwendung des aminofunktionalisierten Styrolderivats gemäß Anspruch 10 oder 11 oder erhalten gemäß Anspruch 9 zur Herstellung von antimikrobiellen Beschichtungen oder Formkörpern.

## Claims

**1.** UP resin composition for the production of products with antimicrobial effect containing

a) an unsaturated polyester from dicarboxylic acid and/or anhydride on the one hand, and diol on the other hand with a molar ratio of 1.25:1 to 0.75:1, with dicarboxylic acid and/or the anhydride having been at least partially functionalized with a radically reactive double bond, and
b) styrene derivative as reactive diluent,
with preferably 0.5 to 8 styrene derivative molecules being present in the composition for each double bond in component a), and
with at least one entity of styrene derivative, dicarboxylic acid and/or diol having been amino-functionalized, with the equation for the amino-functionality being, irrespective of the entity,

$$-(CH_2)_q\text{-}NH_pR^1R^2A_p$$

with

q being either 0, 1 or 2, with $q \neq 0$, provided the functionality is bound to an aromatic,
p being 0 or 1,
$R^1$ having been selected from H, linear or branched or cyclic alkyl residues with 1 to 10 carbon atoms,

$R^2$ being a linear or branched or cyclic alkyl residue with 1 to 10 carbon atoms,
A being the anion of an acid, and
the amine nitrogen N of the above formula being neutral (p=0) or positively (p=1) charged.

**2.** Composition according to claim 1, **characterised in that** the styrene derivative has been amino-functionalized.

**3.** Composition according to claims 1 or 2, **characterised in that** the amino-functionalized styrene derivative is one of these substances,

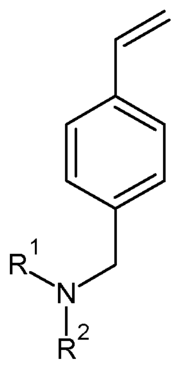

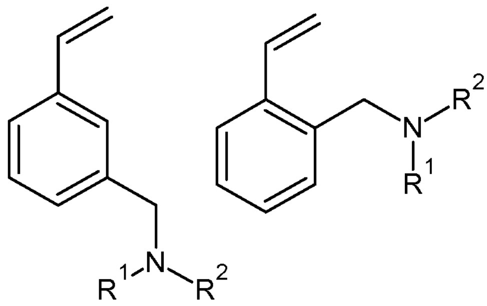

with $R^1$ being a hydrogen, and
$R^2$ having been selected from the group of isopropyl, tert-butyl, tert-pentyl.

**4.** The composition according to any one of claims 1 to 3, **characterised in that** it contains at least 20 wt%, particularly at least 80 wt% of a mixture of components a) and b).

**5.** The composition according to any one of claims 1 to 4, with 1.5 to 4 styrene derivative molecules being contained in the composition for each double bond in component a).

**6.** The use of the composition according to any one of claims 1 to 5 in one of the following processes: coating, varnishing, casting, dipping, laminating, gap impregnation, spinning, gluing, resin injection, pressing, injection moulding, pultrusion, filling and winding.

**7.** Process for the production of cured products, with a composition according to any one of claims 1 to 5 being cured.

**8.** Product with antimicrobial effect containing a cured composition according to claim 7, with more than 20 wt% of the product consisting of components a) and b).

**9.** Use of the product according to claim 8 for the preparation of the following products: Furniture and furniture surfaces, adhesives, veneer and paper laminates, buttons, handles, push-buttons, switches and housings, plates, floorings, tubes, profiles, tanks and containers, in particular for drinking water, food and oil, casing, roof coverings, light panels, sealants, putty, Rawlplug filler, polymer concrete, engineered marble, kitchen sinks, shower basins, bath tubs, wash basins, toilet seats, garden furniture, garden fences, facade plates, cellar window shafts, vehicle parts, lighting support, wind turbines, impregnations, binding agents, casting compounds, filler, and/or reaction mortar, coatings, varnishes, gel coats, top coats, ships, boats, leisure equipment.

**10.** Process for the production of amino-functionalized styrene derivative, wherein the amino functionality is comprised by the formula

$$-(CH_2)_q-NH_pR^1R^2A_p,$$

with

q being either 0, 1 or 2, with $q \neq 0$, provided the functionality is bound to an aromatic,
p being 0 or 1,
$R^1$ having been selected from H, linear or branched or cyclic alkyl residues with 1 to 10 carbon atoms,

$R^2$ being a linear or branched or cyclic alkyl residue with 1 to 10 carbon atoms,
A being the anion of an acid, and
the amine nitrogen N of the above formula being neutral (p=0) or positively (p=1) charged, and

wherein

a) in a first step an aqueous alkali hydroxide solution with a concentration between 3 and 7 mol/l is provided,
b) in a second step an half molar equivalent to one molar equivalent amount of amine with at least one hydrogen atom bound to the nitrogen atom is added to the aqueous alkali hydroxide solution,
c) in a third step 0.2 to 0.5 molar equivalent of halogen alkyl styrene in relation to the amount of alkali hydroxide is added,
d) in a fourth step the resulting reaction solution is further stirred after adding all of the halogen alkyl styrene, over a period of 2 to 48 hours, and
e) in a fifth step the resulting amino-functionalized styrene derivative is separated from the remaining reaction solution.

**11.** Use of one, or a mixture of three substances selected from

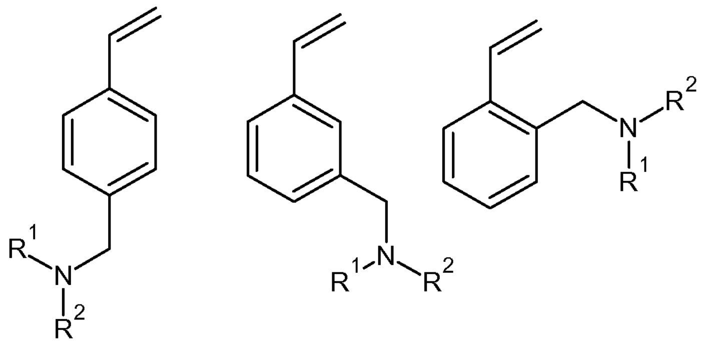

as reactive diluent in resin applications,
with $R^1$ and/or $R^2$ having been independently selected from the group methyl, ethyl, n-propyl, iso-propyl, n-butyl, sec-butyl, iso-butyl, tert-butyl, n-pentyl, 3-pentyl, iso-pentyl, tert-pentyl, cyclopentyl, cyclohexyl,
wherein $R^1$ can also be H.

**12.** Substance selected from

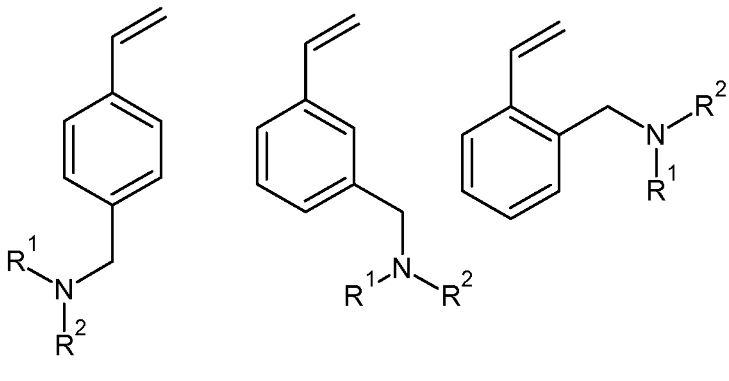

with $R^1$ being hydrogen and $R^2$ being tert-pentyl.

**13.** Use of the amino-functionalized styrene derivative according to claim 10 or 11, or derived according to claim 9, for the production of antimicrobial coatings or mouldings.

## Revendications

1. Une composition de résine UP pour la fabrication de produits ayant une activité antimicrobienne, contenant

   a) un polyester insaturé étant constitué, d'une part, d'acide dicarboxylique et / ou d'anhydride d'acide dicarboxylique, et, d'autre part, de diol dans un rapport molaire de 1,25 : 1 à 0,75 : 1, dans lequel l'acide dicarboxylique et / ou l'anhydride d'acide dicarboxylique sont au moins partiellement fonctionnalisé par une liaison double radicalairement réactive, et
   b) un dérivé de styrène en tant que diluant réactif,
   dans lequel pour chaque double liaison dans le composant a) de 0,5 à 8 molécules dérivées de styrène sont de préférence présents dans la composition, et
   dans lequel au moins une entité choisie parmi un dérivé de styrène, un acide dicarboxylique et / ou un diol est fonctionnalisé par un groupe amine,
   dans lequel la fonctionnalité d'amine, indépendamment de l'entité, tombe sous la formule

   $$-(CH_2)_q-NH_pR^1R^2A_p$$

   dans laquelle

   q est égal à 0, 1 ou 2, ou $q \neq 0$, à condition que la fonctionnalité soit fixé à un noyau aromatique,
   p est égal à 0 ou 1,
   $R^1$ est choisi parmi H, des résidus alkyle linéaires ou ramifiés ou cycliques comportant 1 à 10 atomes de carbone,
   $R^2$ est un résidu alkyle linéaire ou ramifié ou cyclique comportant 1 à 10 atomes de carbone,
   A représente l'anion d'un acide et
   l'azote de l'amine au N de la formule ci-dessus est neutre (p = 0) ou positivement chargé (p = 1).

2. Composition selon la revendication 1, **caractérisé en ce que** le dérivé de styrène est fonctionnalisé par un groupe amine.

3. Composition selon la revendication 1 ou 2, **caractérisé en ce que** le dérivé de styrène fonctionnalisé par un groupe amine est une de ces substances :

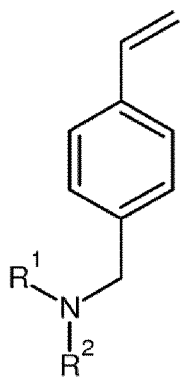
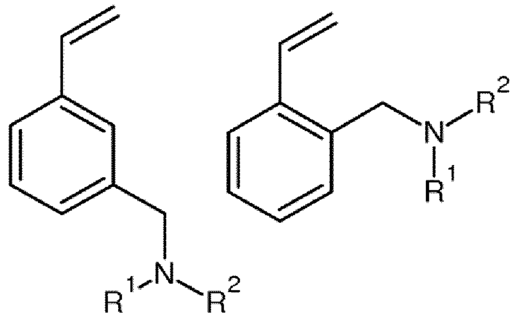

   dans laquelle $R^1$ est un atome d'hydrogène et
   dans laquelle $R^2$ est choisi parmi le groupe constitué par l'iso-propyle, le tert-butyle, le tert-pentyle.

4. Composition selon l'une quelconque des revendications 1 à 3, **caractérisé en ce qu'**elle contient au moins 20% en poids, en particulier au moins 80% en poids d'un mélange des composants a) et b).

5. Composition selon l'une quelconque des revendications 1 à 4, dans laquelle pour chaque double liaison dans le composant a) 1,5 à 4 molécules de dérivé de styrène sont présents dans la composition.

6. Utilisation de la composition selon l'une quelconque des revendications 1 à 5 dans l'une des méthodes de traitement suivantes: le revêtement, la peinture, la coulée, l'immersion, le laminage, l'imprégnation de fente, la centrifugation,

le collage, l'injection de résine, les procédés de pression, le moulage par injection, la pultrusion, le remplissage et l'emballage.

**7.** Procédé pour la fabrication de produits durcis, dans lequel une composition selon l'une quelconque des revendications 1 à 5 est durcie.

**8.** Produit à activité antimicrobienne contenant une composition durcie selon la revendication 7, le produit consistant des composants a) et b) a plus de 20% en poids.

**9.** Utilisation du produit selon la revendication 8 pour la fabrication des produits suivants : des meubles et des revêtements de meubles, des adhésifs, des placages et des stratifiés de papier, des boutons, des poignées, des touches, des interrupteurs et des boîtiers, des panneaux, des parquets, des tubes, des profilés, des réservoirs et des récipients, en particulier pour de l'eau potable, des aliments et des huiles, des doublures, des revêtement de toiture, des panneaux lumineux, des matériaux de scellement, des mastics, des masses a cheviller, du béton de polymère, de l'agglomarbre, des éviers, des receveurs de douche, des baignoires, des éviers, des sièges de toilettes, des meubles de jardin, des clôtures de jardin, des panneaux de façade, des puits de fenêtre en sous-sol, des pièces de véhicule, des support d'éclairage, des éoliennes, des imprégnations, des liants, des agents d'encapsulation, des masses pour mastiquer et / ou du mortier de réaction, des revêtements, des peintures, des enduits gélifiés, des revêtements pardessus, des navires, des bateaux, des articles de loisirs.

**10.** Procédé pour la production d'un dérivé de styrène fonctionnalisé par une amine, dans lequel la fonctionnalité d'amine tombe sous la formule

$$-(CH_2)_q-NH_pR^1R^2A_p$$

dans laquelle

q est égal à 0, 1 ou 2, ou q ≠ 0, à condition que la fonctionnalité soit fixé à un noyau aromatique,
p est égal à 0 ou 1,
$R^1$ est choisi parmi H, des résidus alkyle linéaires ou ramifiés ou cycliques comportant 1 à 10 atomes de carbone,
$R^2$ est un résidu alkyle linéaire ou ramifié ou cyclique comportant 1 à 10 atomes de carbone,
A représente l'anion d'un acide et
l'azote de l'amine au N de la formule ci-dessus est neutre (p = 0) ou positivement chargé (p = 1), et

dans lequel

a) dans une première étape, une solution aqueuse d'hydroxyde de métal alcalin ayant une concentration dans une gamme de 3 à 7 mol / l est prévue,
b) dans une deuxième étape, à cette solution aqueuse d'hydroxyde de métal alcalin un montant demi-équivalent molaire à équivalent molaire d'une amine avec au moins un atome d'hydrogène lié à l'atome d'azote est ajouté,
c) dans une troisième étape, un halogénoalkylestyrène en une quantité de 0,2 à 0,5 équivalents molaires par rapport à la quantité d'hydroxyde de métal alcalin est ajouté,
d) dans une quatrième étape, la solution réactionnelle résultante après l'addition complète de l'halogénoalkylestyrène est encore agité pendant une période dans une plage de 2 à 48 h, et
e) dans une cinquième étape, le dérivé de styrène fonctionnalisé par une amine résultant est séparé de la solution de réactionnelle restante.

**11.** Utilisation d'un ou d'un mélange des trois substances choisi parmi

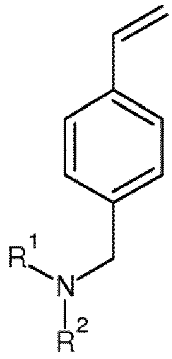 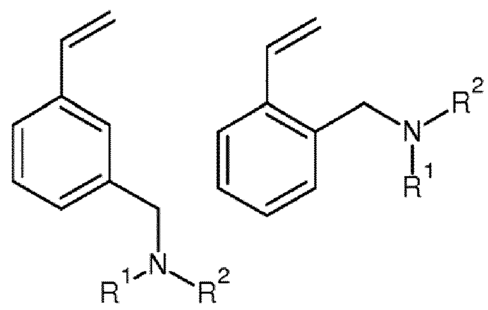

en tant que diluant réactif dans des applications de résine, $R^1$ et / ou $R^2$ étant indépendamment choisis parmi le groupe constitué par le méthyle, l'éthyle, le n-propyle, l'iso-propyle, le n-butyle, le sec-butyle, l'iso-butyle, le tert-butyle, le n-pentyle, le 3-pentyle, l'iso-pentyle, le tert-pentyle, le cyclopentyle, le cyclohexyle, $R^1$ pouvant également être H.

**12.** Substance, choisi parmi

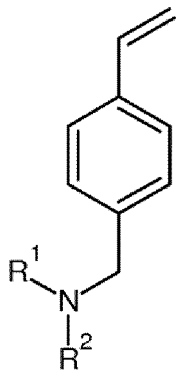 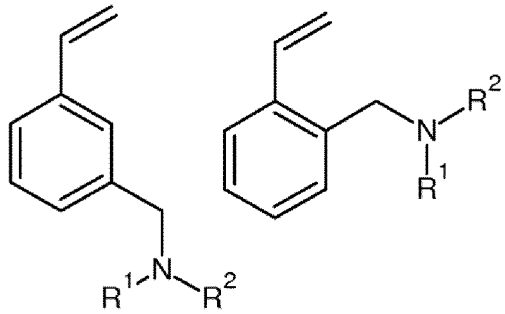

$R^1$ représentant un atome d'hydrogène et $R^2$ représentant du tert-pentyle.

**13.** Utilisation du dérivé de styrène fonctionnalisé par une amine selon la revendication 10 ou 11 ou obtenus selon la revendication 9 pour la préparation de revêtements ou de corps moulés antimicrobiens.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6242526 B
- US 4810567 A
- US 5614568 A
- US 4447580 A
- JP 2000239281 A
- US 6200680 B
- US 4021416 A
- DE 10242561 A1
- DE 4432985 A1
- DE 19723504 C1


### In der Beschreibung aufgeführte Nicht-Patentliteratur


- **KUNO et al.** *Reactive & Functional Polymers,* 2000, vol. 43, 43-51
- **H. MENZEL.** Schutzschicht gegen Bakterien. *Nachrichten aus der Chemie,* November 2011, vol. 59, 1039-43